# FASCICULE DE BREVET EUROPEEN

(11) **EP 3 013 842 B1**
(45) Date de publication et mention de la délivrance du brevet: **04.03.2020**
(21) Numéro de dépôt: 14731805.9
(22) Date de dépôt: 28.05.2014
(51) Int. Cl.: C07H 15/12, A01N 43/16, C07H 15/26, A61K 31/7032, A61P 31/00, C07C 215/10

(54) **NOUVEAU PROCÉDÉ DE SYNTHÈSE DE DÉRIVÉS N-ALKYL-GLYCOSYL(DI)AMINE ET LEURS UTILISATIONS CONTRE LES PHYTOPATHOGÈNES**
NEUARTIGES VERFAHREN ZUR SYNTHESE VON N-ALKYL-GLYCOSYL(DI)AMIN-DERIVATEN UND VERWENDUNGEN DAVON GEGEN PHYTOPATHOGENE
NOVEL METHOD FOR SYNTHESISING N-ALKYL-GLYCOSYL(DI)AMINE DERIVATIVES AND USES OF SAME AGAINST PHYTOPATHOGENS

(30) Priorité: 31.05.2013 FR 1355024
(43) Date de publication de la demande: 04.05.2016
(73) Titulaire: Sipre, 62217 Achicourt (FR); Université de Picardie Jules Verne, 80025 Amiens Cedex 1 (FR)
(72) Inventeur: EPOUNE LINGOME, Cédric, F-80000 Amiens (FR); WADOUACHI, Anne, F-80136 Rivery (FR); POURCEAU, Gwladys, F-72210 Louplande (FR); BEURY, Amélie, F-62217 Beaurains (FR); GOBERT-DEVEAUX, Virginie, Faches Thumesni 59155 (FR)
(74) Mandataire: Gevers & Orès
(86) Numéro de dépôt international: PCT/IB2014/061779
(87) Numéro de publication internationale: WO 2014/195828

(56) Documents cités:
- WO-A1-01/05224
- MUHIZI T ET AL: "Synthesis and evaluation of N-alkyl-beta-d-glucosylamines on the growth of two wood fungi, Coriolus versicolor and Poria placenta", CARBOHYDRATE RESEARCH, vol. 343, no. 14, 22 septembre 2008 (2008-09-22), pages 2369-2375, XP025474140, PERGAMON, GB ISSN: 0008-6215, DOI: 10.1016/J.CARRES.2008.07.005 [extrait le 2008-07-15]
- VIRGINIE NETO ET AL: "-glycosylamine Derivatives on Antifungal Properties", JOURNAL OF AGRICULTURAL AND FOOD CHEMISTRY, vol. 60, no. 42, 24 octobre 2012 (2012-10-24), pages 10516-10522, XP055080352, ISSN: 0021-8561, DOI: 10.1021/jf3015798
- T. MUHIZI ET AL: "-glucosylamines and Their Antimicrobial Activity against Fusarium proliferatum , Salmonella typhimurium , and Listeria innocua", JOURNAL OF AGRICULTURAL AND FOOD CHEMISTRY, vol. 57, no. 23, 9 décembre 2009 (2009-12-09), pages 11092-11099, XP055080357, ISSN: 0021-8561, DOI: 10.1021/jf9016114
- CHUPAKHINA T A ET AL: "Synthesis and investigation of antimicrobial activity of 8-hydroxyquinoline glucosaminides", RUSSIAN JOURNAL OF BIOORGANIC CHEMISTRY, vol. 38, no. 4, 14 juillet 2012 (2012-07-14), pages 422-427, XP035085331, SP MAIK NAUKA/INTERPERIODICA, DORDRECHT ISSN: 1608-330X, DOI: 10.1134/S106816201204005X
- IDDON L ET AL: "Syntheses and structures of anomeric quaternary ammonium beta-glucosides and comments on the anomeric C-N bond lengths", TETRAHEDRON, vol. 65, no. 32, 8 août 2009 (2009-08-08), pages 6396-6402, XP026281503, ELSEVIER SCIENCE PUBLISHERS, AMSTERDAM, NL ISSN: 0040-4020, DOI: 10.1016/J.TET.2009.05.086 [extrait le 2009-06-06]
- James Mack and Sivaranakrishnan Muthukrishnan: "Solvent-Free Synthesis"; "Chapter 11" In: "Green Techniques for Organic Synthesis and Medicinal Chemistry", 2012, John Wiley & Sons, Ltd., XP002713483, pages 297-323, pages 308-309
- I. RICO-LATTES AND A. LATTES: "Synthesis of new sugar-based surfactants having biological applications: key role of their self-association", COLLOIDS AND SURFACES A: PHYSICOCHEMICAL AND ENGINEERING ASPECTS, vol. 123-124, 1997, pages 37-48, XP5095617,

## Description

La présente invention a pour objet un nouveau procédé de synthèse sans solvant de dérivés *N*-alkyl-glycosyl(di)amine répondant à la formule générale (I) suivante :

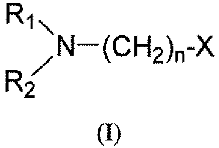

L'invention concerne également l'utilisation de dérivés *N*-alkyl-glycosyl(di)amine répondant à la formule générale (I) comme agents antibactériens et/ou antifongiques contre des phytopathogènes. Enfin, l'invention concerne l'utilisation de sels *N*-alkyl-glycosyl(di)ammonium quaternaires répondant à la formule générale (II) et de dérivés *N-*alkyl-glycamine répondant à la formule générale (III) obtenus à partir de dérivés *N*-alkyl-glycosyl(di)amine répondant à la formule générale (I) comme agents antibactériens et/ou antifongiques contre des phytopathogènes.

Depuis plusieurs années, la synthèse de molécules bioactives à grand spectre d'action (antibactériens, antifongiques, antivirales, nématicides, éliciteurs de défenses naturelles), susceptibles d'inhiber et/ou d'éradiquer les phytopathogènes de plantes telles que la pomme de terre, la tomate, la vigne ou la betterave, a connu un intérêt grandissant. En effet, la synthèse de telles molécules revêt des enjeux économiques et environnementaux de premier ordre, ces molécules présentant une très haute valeur ajoutée et une éco-toxicité réduite, tout en satisfaisant aux critères de biocompatibilité et de biodégradabilité. La chimie mise en œuvre pour la synthèse de ces molécules doit donc être une chimie à développement durable, régie par des principes tels que l'économie d'atomes, la conception de produits chimiques moins nocifs et plus sécuritaires, l'utilisation de solvants et auxiliaires plus sécuritaires, l'amélioration des rendements énergétiques, l'utilisation de matières premières renouvelables tels que les hydrates de carbones (sucres), la réduction de la quantité de produits dérivés, la catalyse.

La présente invention s'inscrit dans cette lignée. Elle concerne un nouveau procédé de synthèse, dit « procédé par chimie verte », de dérivés *N*-alkyl-glycosyl(di)amine utilisés comme biocides contre des phytopathogènes. Des procédés de préparation de dérivés *N-*(dialkylamino)alkyl-glycosylamine ont en effet déjà été décrits dans l'art antérieur (Demandes FR 2 767 134, FR 2 661 413 et FR 2 440 159). D'autres procédés similaires sont également décrits par Muhizi T. et al. (Carb. Res. 2008, vol. 343(14) pp.2369-75), Neto V. et al. (J. Agri. Food Chem. 2012, vol. 60(42) pp. 11092-9), Muhizi T. et al. (J. Agri. Food Chem. 2009, vol. 57(23) pp. 422-7). Toutefois, ces synthèses ont toujours été réalisées en milieu solvant.

Les dérivés *N-*alkyl-glycosyl(di)amine obtenus selon le procédé de l'invention sont des composés « bio-sourcés », potentiellement moins toxiques que les pesticides habituellement utilisés, et susceptibles de lutter contre les phytopathogènes présents dans les sols, telles que les bactéries à Gram-négatif responsables par exemple de la galle du collet induit par l'agent pathogène *Agrobacterium tumefaciens,* ou de la jambe noire chez la pomme de terre induite par les bactéries des genres *Pectobacterium* et *Dickeya.* En effet, il n'existe aucun moyen de lutte chimique contre la maladie de la jambe noire qui affecte les champs de pomme de terre, ainsi que les tubercules en cours de stockage, seules des méthodes prophylactiques veillant à limiter leur dissémination sont aujourd'hui utilisées.

Les inventeurs se sont donc donnés pour but de concevoir un procédé de synthèse de dérivés *N*-alkyl-glycosyl(di)amine par mécanosynthèse, plus sécuritaire que les procédés habituellement utilisés car n'utilisant pas de solvant organique nocif, ledit procédé conduisant à de très bons rendements. Les procédés par mécanosynthèse, auparavant destinés à la préparation de composés inorganiques, peuvent donc aujourd'hui être adaptés à la synthèse de composés glycosylés.

Ainsi, le premier objet de l'invention concerne un procédé de synthèse sans solvant de dérivés *N*-alkyl-glycosyl(di)amine répondant à la formule générale (I) suivante : 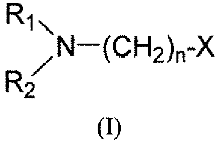 dans laquelle :
- R₁ est un radical glycosyle, et de préférence un résidu mono, oligo ou polysaccharidique,
- R₂ représente un atome d'hydrogène, un radical glycosyle, ou un radical alkyle en C₁-C₂₂,
- n est un nombre entier allant de 0 à 22,
- X représente un atome d'halogène, un radical alkyle, alcényle, alcynyle, un groupe hydroxyle, un groupe carboxyle ou carbaldéhyde, un groupe aryle ou hétéroaryle, ou un groupe -NR₃R₄, dans lequel R₃ représente un atome d'hydrogène ou un radical glycosyle, et de préférence un résidu mono, oligo ou polysaccharidique, et R₄ représente un atome d'hydrogène ou un radical alkyle en C₁-C₁₂,
   à la condition que n soit différent de 0 lorsque X est un groupe -NR₃R₄,
ledit procédé comprenant les étapes suivantes :
(i) le mélange dans un réacteur, de préférence dans un vibro-broyeur ou dans un réacteur pour broyage à billes à mouvement vibrationnel ou à mouvement planétaire, d'un composé ose ou oside et d'une amine de formule R₂NH-(CH₂)ₙ-X, et
(ii) le broyage dudit mélange dans un broyeur à billes.

Au sens de la présente invention, on entend par :
- Alkyle : un groupe aliphatique hydrocarboné saturé, linéaire ou ramifié, pouvant avoir de 1 à 22 atomes de carbone, de préférence 1 à 12 atomes de carbone, et encore plus préférentiellement 1 à 6 atomes de carbone. Le terme « ramifié » signifie qu'au moins un groupe alkyle inférieur tel qu'un méthyle ou un éthyle est porté par une chaîne alkyle linéaire. A titre de groupe alkyle, on peut mentionner par exemple les groupes méthyle, éthyle, *n-*propyle, *i*-propyle, *n*-butyle, *t*-butyle et *n*-pentyle, *n*-hexyle, *n*-heptyle, *n*-octyle, *n*-nonyle, *n-*décyle, *n*-undécyle, *n*-dodécyle ;
- Alcényle : un groupe aliphatique hydrocarboné, linéaire ou ramifié, possédant une insaturation caractérisée par la présence d'une double liaison carbone-carbone. L'alcène a de préférence 2 à 12 atomes de carbone, et encore plus préférentiellement 2 à 6 atomes de carbone ;
- Alcynyle : un groupe aliphatique hydrocarboné, linéaire ou ramifié, possédant une insaturation caractérisée par la présence d'une triple liaison carbone-carbone. L'alcyne a de préférence 2 à 12 atomes de carbone, et encore plus préférentiellement 2 à 6 atomes de carbone ;
- Aryle : tout groupe fonctionnel ou substituant dérivé d'au moins un cycle aromatique ; un cycle aromatique correspond à tout groupe mono- ou polycyclique plan comportant un système *π* délocalisé dans lequel chaque atome du cycle comporte une orbitale p, lesdites orbitales p se recouvrant les unes les autres ; parmi de tels groupes aryles, on peut mentionner les groupes phényle, biphényle, naphthalène et anthracène. Les groupes aryles de l'invention comprennent de préférence de 6 à 22 atomes de carbone, de manière préférée de 6 à 12 atomes de carbone, et de manière encore plus préférée 5 ou 6 atomes de carbone ;
- Hétéroaryle : tout groupe fonctionnel ou substituant dérivé d'au moins un cycle aromatique tel que défini ci-dessus et contenant au moins un hétéroatome choisi parmi P, S, O et N ; parmi les groupes hétéroaryles, on peut mentionner les groupes furane, pyridine, pyrrole, thiophène, imidazole, pyrazole, oxazole, isoxazole, thiazole, triazole, tétrazole, pyridine, pyrazine, pyrimidine, pyridazine, benzofurane, isobenzofurane, indole, isoindole, benzothiophène, benzo[*c*]thiophène, benzimidazole, indazole, benzoxazole, benzisoxazole, benzothiazole, quinoléine, isoquinoléine, quinoxaline, quinazoline, cinnoline, purine, et acridine. Les groupes aryles de l'invention comprennent de préférence de 4 à 22 atomes de carbone, de manière préférée de 4 à 12 atomes de carbone, et de manière encore plus préférée 5 ou 6 atomes de carbone ;
- Les atomes d'halogène sont choisis parmi les atomes de brome, de chlore, de fluore et d'iode, et de préférence parmi les atomes de brome, de chlore et d'iode.

Le composé ose ou oside de l'invention est un saccharide choisi parmi un radical rhamnosyle, glucosyle, galactosyle, maltosyle et lactosyle.

Le procédé de l'invention peut comprendre une étape préalable au cours de laquelle l'amine de formule R₂NH-(CH₂)ₙ-X est mélangée à un auxiliaire, de préférence à l'aide d'un mortier, ledit auxiliaire pouvant être neutre ou basique et choisi parmi la silice (SiO₂), l'alumine (Al₂O₃), les argiles et les carbonates. Cette étape préalable de mélange avec un auxiliaire améliore de façon quantitative le rendement de la synthèse, les inventeurs ayant observé une amélioration des rendements de 40-60% à 75-97% lors d'un mélange préalable de l'amine avec un auxiliaire.

On entend par auxiliaire un agent qui permet une meilleure dispersion des poudres. Il peut être inerte chimiquement ou non, et être choisi parmi la silice, l'alumine, les argiles telles que les argiles montmorillonite K10 ou KSF, et les carbonates tels que les carbonates de sodium ou de potassium.

Au cours de l'étape (i), le rapport molaire du composé ose ou oside à l'amine peut être compris entre 1/1 et 1/1,2.

L'étape (ii) de broyage peut durer au moins 1 heure, et de préférence de 1 à 4 heures. Au cours de cette étape (ii), le broyeur fonctionne de préférence à une fréquence comprise entre 20 et 50 Hz, et de préférence à 50 Hz.

Selon un mode de réalisation préféré, le réacteur et/ou les billes du broyeur sont en inox, en carbure de tungstène ou en zircone. Le réacteur et les billes du broyeur sont de préférence constitués du même matériau.

Selon un mode de réalisation particulièrement préféré, les billes du broyeur et le mélange du composé ose ou oside et de l'amine de formule R₂NH-(CH₂)ₙ-X représentent au moins 1/3 du volume du réacteur.

Le procédé de l'invention peut également comprendre une étape (iii) supplémentaire de purification et/ou de filtration. Lorsque le procédé de l'invention est réalisé sans étape d'imprégnation préalable, l'étape (iii) est de préférence une étape de purification sur silice. Lorsque le procédé de l'invention est réalisé avec une étape préalable d'imprégnation, l'étape (iii) est de préférence une étape de filtration sur silice, sur colonne ou fritté.

Selon une première alternative, le procédé de l'invention peut comprendre une étape (iv) subséquente de transformation du dérivé *N*-alkyl-glycosyl(di)amine de formule (I) en un sel *N*-alkyl-glycosyl(di)ammonium quaternaire par réaction avec un agent de quaternisation de formule R₅-Y, dans lequel :
- R₅ représente un atome d'hydrogène ou un radical alkyle en C₁-C₂₂, et
- Y représente un atome d'halogène, un groupe sulfate R₆O-SO₂-O- ou un groupe sulfonate R₆-SO₂-O-, dans lesquels R₆ est un radical alkyle en C₁-C₁₂, les groupes sulfonates pouvant être des groupes triflate, tosylate, mésylate ou nosylate.

Selon un mode de réalisation préféré, le sel *N*-alkyl-glycosyl(di)ammomum quaternaire de l'étape (iv) est obtenu après réaction du dérivé *N*-alkyl-glycosyl(di)amine avec un excès d'agent de quaternisation dans un vibro-broyeur ou un réacteur pour broyage à billes à mouvement vibrationnel ou planétaire.

Le sel *N*-alkyl-glycosyl(di)ammonium quaternaire obtenu répond à la formule (II) suivante : 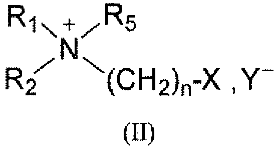 dans laquelle R₁, R₂, n et X sont tels que définis précédemment, et :
- R₅ représente un atome d'hydrogène ou un radical alkyle en C₁-C₂₂, et
- Y représente un ion halogénure, un ion sulfate R₆O-SO₂-O⁻ ou un ion sulfonate R₆-SO₂-O⁻, dans lesquels R₆ est un radical alkyle en C₁-C₁₂, les ions sulfonates pouvant être des ions triflate, tosylate, mésylate ou nosylate,
à la condition que n soit différent de 0 lorsque X est un groupe -NR₃R₄.

L'excès d'agent de quaternisation peut être compris entre 1,5 et 10 équivalents molaires par rapport au dérivé *N*-alkyl-glycosyl(di)amine de formule (I), et de préférence entre 2,5 et 5 équivalents molaires.

Lors de l'étape (iv), le broyage peut durer au moins 2h, et de préférence 7h.

La purification du sel *N*-alkyl-glycosyl(di)ammonium quaternaire de formule (II) peut être réalisée selon un procédé comprenant une étape d'acétylation, suivie d'une étape d'évaporation et d'une étape de décantation avec un solvant organique et de l'eau distillée. Au cours de l'étape d'acétylation, l'ion Y⁻ peut se transformer en ion carboxylate R₆-COO⁻, dans lesquels R₆ est un radical alkyle en C₁-C₁₂. Enfin, une étape de lyophilisation ou de séchage de la phase aqueuse, et une étape de filtration sur gel de silice, peuvent être réalisées pour isoler le sel *N*-alkyl-glycosyl(di)ammonium quaternaire de formule (II).

De manière préférée, la purification du sel *N*-alkyl-glycosyl(di)ammonium quaternaire de formule (II) est réalisée selon un procédé comprenant une étape d'acétylation mettant en œuvre un mélange d'anhydride acétique avec un composé acide ou un mélange d'anhydride acétique avec un composé basique ou un mélange de chlorure d'acétyle avec un composé basique. Le mélange préféré utilisé pour l'acétylation est le mélange d'anhydride acétique et de triflate de cérium.

Selon une seconde alternative, le procédé de l'invention peut comprendre une étape (iv') subséquente de transformation du dérivé N-alkyl-glycosyl(di)amine de formule (I) en un dérivé *N*-alkyl-glycamine par broyage du dérivé *N*-alkyl-glycosyl(di)amine de formule (I) avec un agent de réduction et un acide, en présence d'un solvant polaire.

Le dérivé *N*-alkyl-glycamine obtenu répond à la formule (III) suivante : 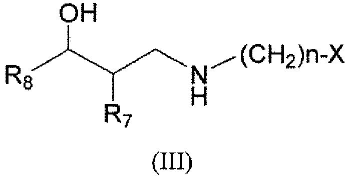 dans laquelle n et X sont tels que définis précédemment, et :
- R₇ représente un atome d'hydrogène, un groupe -OH ou -NH₂, et
- R₈ représente un atome d'hydrogène ou un groupe -CH(OR₉)R₁₀ dans lequel R₉ représente un atome d'hydrogène ou un radical glycosyle, et de préférence un résidu mono, oligo ou polysaccharidique, et R₁₀ représente un atome d'hydrogène, un groupe
- CH₂OH ou -CH(OH)R₁₁ dans lequel R₁₁ représente un groupe -CH₂OH ou un radical alkyle en C₁-C₄, et de préférence un groupe méthyle.

L'étape (iv') de broyage est de préférence réalisée dans un réacteur, et encore plus préférentiellement dans un vibro-broyeur ou dans un réacteur pour broyage à billes à mouvement vibrationnel ou à mouvement planétaire.

L'agent de réduction est avantageusement choisi parmi les borohydrures métalliques tels que NaBH₄, LiBH₄, NaBH₃CN, LiBH₃CN, NaBH(OAc)₃, LiBH(OAc)₃, ZnBH₄, le L-sélectride de formule C₁₂H₂₇BLi, le diborane B₂H₆, ou un hydrure d'aluminium choisi parmi LiAlH₄, LiAlH(O-*t*Bu)₃, l'hydrure de diisobutylaluminium (ou DIBAL) de formule C₈H₁₉Al, l'hydrure de bis(2-méthoxyéthoxy)aluminum de sodium (ou Red-Al) de formule C₆H₁₆AlNaO₄, ou les hydrures covalents tels que GaH₃, NaGaBH₄. L'agent de réduction préféré est NaBH₄.

Au sens de la présente invention, on entend par acide un acide de Lewis ou un acide de Brönsted, de préférence un acide de Brönsted porteur d'une fonction acide carboxylique ou acide sulfonique. De manière avantageuse, l'acide mis en œuvre est l'acide para-toluène sulfonique ou l'acide benzoïque.

Le broyage est de préférence réalisé en présence d'une quantité catalytique de solvant polaire. Le pourcentage en poids de solvant polaire par rapport au poids total des réactifs peut varier de 10 à 50% en poids, et de préférence entre 10 et 20% en poids. Le solvant polaire peut être choisi parmi le diméthylsulfoxyde (DMSO), le diméthylformamide (DMF), l'acétonitrile, l'éthanol, le méthanol, le butanol, le propanol, l'isopropanol, l'eau et l'acétone, les solvants polaires préférés étant le méthanol et l'éthanol.

Selon un mode de réalisation avantageux, l'étape (iv') de broyage peut comprendre un broyage préalable du dérivé *N*-alkyl-glycosyl(di)amine de formule (I) avec l'agent de réduction et l'acide, suivie de l'ajout du solvant polaire, puis d'une nouvelle étape de broyage cette fois en présence du solvant polaire. Le broyage préalable peut durer de 1 à 30 minutes, de préférence de 5 à 15 minutes, et encore plus préférentiellement 10 minutes. Le broyage en présence du solvant polaire peut durer de 5 à 30 minutes, de préférence de 10 à 20 minutes, et encore plus préférentiellement 15 minutes.

La purification du dérivé *N*-alkyl-glycamine de formule (III) peut être réalisée selon un procédé comprenant une étape de quaternisation, suivie d'une étape de filtration et d'une étape de régénération de l'amine sur une résine échangeuse d'ions dans un solvant organique. Enfin une étape de filtration, suivie d'une étape d'évaporation, peuvent être réalisées pour isoler le dérivé *N*-alkyl-glycamine de formule (III).

L'étape de quaternisation est de préférence réalisée dans un solvant organique avec un acide tel que HCl, HBr, H₃PO₄, H₂SO₄ et HNO₃, et de préférence avec HCl.

L'étape de régénération de l'amine est de préférence réalisée sur une résine échangeuses d'anions choisie parmi les résines anioniques porteuses de groupes aminés tertiaires ou quaternaires, et de préférence sur une résine échangeuse d'anions Amberlite IRA 402-Cl⁻.

Il est également décrit l'utilisation de dérivés *N*-alkyl-glycosyl(di)amines répondant à la formule générale (I) suivante : 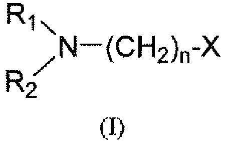 dans laquelle :
- R₁ est un radical glycosyle, de préférence un résidu mono, oligo ou polysaccharidique,
- R₂ représente un atome d'hydrogène, un radical glycosyle, ou un radical alkyle en C₁-C₂₂,
- n est un nombre entier allant de 0 à 22,
- X représente un atome d'halogène, un radical alkyle, alcényle, alcynyle, un groupe hydroxyle, un groupe carboxyle ou carbaldéhyde, un groupe aryle ou hétéroaryle, ou un groupe -NR₃R₄, dans lequel R₃ représente un atome d'hydrogène ou un radical glycosyle, de préférence un résidu mono, oligo ou polysaccharidique, et R₄ représente un atome d'hydrogène ou un radical alkyle en C₁-C₁₂,
à la condition que n soit différent de 0 lorsque X est un groupe -NR₃R₄, comme agents antibactériens et/ou antifongiques contre les phytopathogènes suivants : *Agrobacterium tumefaciens, Pectobacterium atrosepticum, Dickeya dianthicola, Fusarium sambucinum, Fusarium roseum* et *Phytophthora infestans,* les phytopathogènes *Pectobacterium atrosepticum, Dickeya dianthicola, Fusarium sambucinum, Fusarium roseum* et *Phytophthora infestans* étant des phytopathogènes de pommes de terre.

il est également décrit l'utilisation de sels *N*-alkyl-glycosyl(di)ammonium quaternaires répondant à la formule générale (II) suivante : 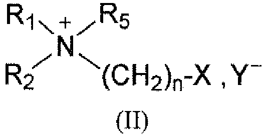 dans laquelle R₁, R₂, n et X sont tels que définis précédemment, et :
- R₅ représente un atome d'hydrogène ou un radical alkyle en C₁-C₂₂, et
- Y représente un ion halogénure, un ion sulfate R₆O-SO₂-O⁻ ou un ion sulfonate R₆-SO₂-O⁻, dans lesquels R₆ est un radical alkyle en C₁-C₁₂, les ions sulfonates pouvant être des ions triflate, tosylate, mésylate ou nosylate,
à la condition que n soit différent de 0 lorsque X est un groupe -NR₃R₄, comme agents antibactériens et/ou antifongiques contre les phytopathogènes, notamment contre les phytopathogènes tels que : *Agrobacterium tumefaciens, Pectobacterium atrosepticum, Dickeya dianthicola, Fusarium sambucinum, Fusarium roseum* et *Phytophthora infestans,* les phytopathogènes *Pectobacterium atrosepticum, Dickeya dianthicola, Fusarium sambucinum, Fusarium roseum* et *Phytophthora infestans* étant des phytopathogènes de pommes de terre.

Enfin, est également décrit l'utilisation de dérivés *N*-alkyl-glycamine répondant à la formule générale (III) suivante : 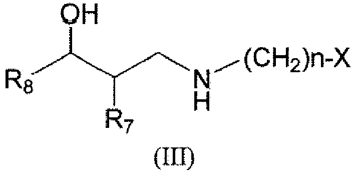 dans laquelle n et X sont tels que définis précédemment, et :
- R₇ représente un atome d'hydrogène, un groupe -OH ou -NH₂, et
- R₈ représente un atome d'hydrogène ou un groupe -CH(OR₉)R₁₀ dans lequel R₉ représente un atome d'hydrogène ou un radical glycosyle, et de préférence un résidu mono, oligo ou polysaccharidique, et R₁₀ représente un atome d'hydrogène, un groupe -CH₂OH ou -CH(OH)R₁₁ dans lequel R₁₁ représente un groupe -CH₂OH ou un radical alkyle en C₁-C₄, et de préférence un groupe méthyle,
comme agents antibactériens et/ou antifongiques contre les phytopathogènes suivants : *Agrobacterium tumefaciens, Pectobacterium atrosepticum, Dickeya dianthicola, Fusarium sambucinum, Fusarium roseum* et *Phytophthora infestans,* les phytopathogènes *Pectobacterium atrosepticum, Dickeya dianthicola, Fusarium sambucinum, Fusarium roseum* et *Phytophthora infestans* étant des phytopathogènes de pommes de terre.

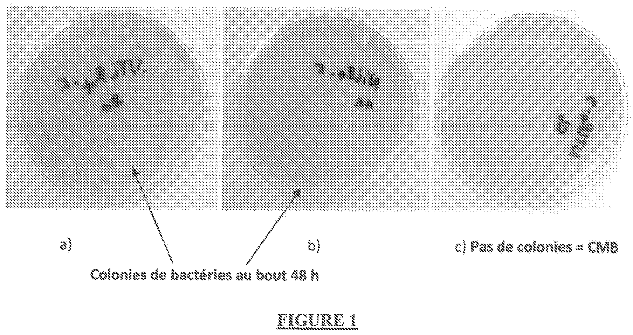

Outre les dispositions qui précèdent, l'invention comprend encore d'autres dispositions qui ressortiront du complément de description qui suit, qui se rapporte à des exemples de mise en œuvre du procédé de l'invention pour la synthèse de dérivés *N*-alkyl-glycosyl(di)amine répondant à la formule générale (I), de sels *N*-alkyl-glycosyl(di)ammonium quaternaires répondant à la formule générale (II), et de dérivés *N*-alkyl-glycamine répondant à la formule générale (III), ainsi qu'à la **Figure 1** annexée qui illustre l'activité antibactérienne de dérivés *N*-alkyl-glycosylamine de formule (I) et de dérivés *N*-alkyl-glycamines de formule (III) contre *Pectobacterium atrosepticum, Agrobacterium tumefaciens,* et *Dickeya dianthicola.*

### MATÉRIEL ET MÉTHODES :

### Caractérisation :

Les produits décrits dans les exemples ci-après ont été caractérisés par chromatographie sur couche mince sur des plaques en verre ou d'aluminium MERCK recouverte d'un gel de silice 60 F₂₅₄ de 0,25 mm d'épaisseur pour la phase directe. La révélation a été réalisée par pulvérisation d'une solution de ninhydrine ou de molybdate de cérium, suivi d'un chauffage de la plaque.

Les purifications par chromatographie sur colonne ont été effectuées en phase liquide sur colonne ouverte. La phase stationnaire utilisée est le gel de silice Merck 60 (70-230 mesh ASTM) (0,063-0,200 µm).

Les spectres de RMN du proton (¹H) et du carbone (¹³C) ont été réalisés sur des spectromètres Bruker Advance 300. Les valeurs des déplacements chimiques (δ) sont exprimées en partie par million (ppm). Les constantes de couplage J sont exprimées en Hertz (Hz). La multiplicité des signaux est indiquée à l'aide des abréviations suivantes :
s = singulet d = doublet dd= doublet de doublet t = triplet m = multiplet

Les spectres de masse ont été réalisés sur un spectromètre de masse de type Q-Tof Tandem en polarité positive. Les échantillons sont ionisés par électrospray avec détection RID. D'autres spectres ont été effectués sur un spectromètre WATERS ZQ en mode électrospray également. Les composés ont été préalablement dissous dans du méthanol.

### Exemple 1 : Préparation d'un composé la de formule :

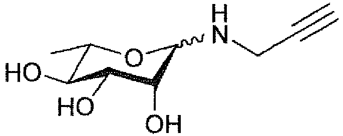

De la propargylamine (0,71 g; 2,5 mmol) est mélangée à 3 g d'alumine neutre (Al₂O₃). Les réactifs sont broyés dans un mortier à l'aide d'un pilon pendant 2 minutes, puis 2,21 g de L-rhamnose monohydrate (12,2 mmol) sont ajoutés dans le mortier. Le mélange ainsi formé est broyé à l'aide d'un pilon pendant 2 minutes, puis introduit dans une jarre en inox munie de 4 billes en inox de 13 mm de diamètre. La jarre est placée dans un broyeur à billes type vibrationnel Spex 8000M et agitée pendant 1h30.

Le brut réactionnel est dissous à reflux dans 200 mL d'éthanol, puis filtré sur un fritté comportant 50 g de silice et lavé avec 100 mL d'éthanol chaud. Après évaporation de l'éthanol sous pression réduite, un solide jaune est obtenu.

Un lavage avec 10 mL de diéthyl éther à 40°C permet d'obtenir 2,3 g (94%) d'un composé **la** sous forme d'une poudre jaune-pâle.

Mélange d'isomères α, β : α/β : 88/12 ;

Isomère α : RMN ¹H (CD₃OD) : *δ* 4.18 (d, 1H, *J* = 1.1 Hz, H-1) ; 3.79 (dd, 1H, *J* = 3.4, 1.0 Hz, H-2) ; 3.61 (dd, 1H, *J* = 16.4, 2.6 Hz, -HN-***CH₂***-) ; 3.52 (dd, 1H, *J* = 16.4, 2.6 Hz, -HN-***CH₂***-) ; 3.42 (dd, 1H, *J* = 9.2, 3.3 Hz, H-3) ; 3.29 (t, 1H, *J* = 9.2 Hz, H-4) ; 3.18 (dq, 1H *J* = 9.2, 6.0 Hz, H-5) ; 2.57 (t, 1H, *J* = 2.5 Hz, ***HC*≡**C-) ; 1.28 (d, 3H, *J* = 6.1 Hz, H-6).

RMN ¹³C (CD₃OD) : *δ* 86.7 (C-1), 82.1 (HC≡***C***-) ; 75.8 (C-3), 74.6 (C-5), 74.0 (C-4), 72.8 (C-2 + ***HC*≡**C-), 34.6 (-HN-***CH₂***-), 18.1 (C-6).

Spectrométrie de masse haute résolution HRMS [M+H]⁺ calculée pour C₉H₁₅NO₄ : *m*/*z* 202.1035 trouvée *m*/*z* 202.1070.

### Exemple 2 : Préparation d'un composé Ib de formule :

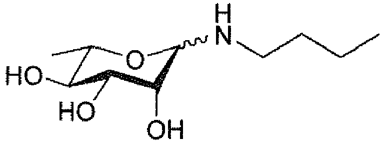

De la butylamine (0,45 g ; 6,1 mmol) est mélangée à 1,5 g de silice (SiO₂). Les réactifs sont broyés dans un mortier à l'aide d'un pilon pendant 2 minutes, puis 1 g de L-rhamnose monohydrate (5,5 mmol) est ajouté dans le mortier. Le mélange ainsi formé est broyé à l'aide d'un pilon pendant 2 minutes, puis introduit dans une jarre en inox munie de 4 billes en inox de 13 mm de diamètre. La jarre est placée dans un broyeur à billes type vibrationnel Spex 8000M et agitée pendant lh30.

Le brut réactionnel est dissous à reflux dans 100 mL d'éthanol, puis filtré sur un fritté comportant 50 g de silice et lavé avec 50 mL d'éthanol chaud. Après évaporation de l'éthanol sous pression réduite, un solide blanc-cassé est obtenu.

Un lavage avec 10 mL de diéthyl éther à 40°C, suivi d'une filtration sur fritté, permet d'obtenir 1,15g (86%) d'un composé **Ib** sous forme de poudre blanche.

Mélange d'isomères α, β : α/β : 94/6 ;

Isomère α : RMN ¹H (CD₃OD) : *δ* 4.04 (d, 1H, *J* = 1.1 Hz, H-1) ; 3.78 (dd, 1H, *J* = 3.3, 1.1 Hz, H-2) ; 3.40 (dd, 1H, *J* = 9.2, 3.3 Hz, H-3) ; 3.28 (t, 1H, *J* = 9.2 Hz, H-4) ; 3.17 (dq, *J* = 9.1, 6.0 Hz, 1H, H-5) ; 2.93 (ddd, 1H, *J* = 11.6, 8.3, 6.5 Hz, -HN-***CH₂***-) ; 2.59 (ddd, 1H, *J* = 11.6, 8.4, 5.9 Hz, -HN-***CH₂***-) ; 1.58-1.30 (m, 4H, -CH₂-CH₂-) ; 1.27 (d, 3H, *J* = 6.0 Hz, H-6) ; 0.94 (t, 3H, *J* = 7.2 Hz, ***CH₃***-CH₂).

RMN ¹³C (CD₃OD) : *δ* 88.4 (C-1), 76.0 (C-3), 74.7 (C-5), 74.1 (C-4), 73.1 (C-2), 46.0 (-HN-***CH₂***-), 33.2 (-HN-CH₂-***CH₂***-), 21.5 (-***CH₂***-CH₃), 18.1 (C-6), 14.3 (***CH₃***-CH₂).

Spectrométrie de masse haute résolution HRMS [M+H]⁺ calculée pour C₁₀H₂₁NO₄ : *m*/*z* 220.1549, trouvée *m*/*z* 220,1546.

### Exemple 3 : Préparation d'un composé Ic de formule :

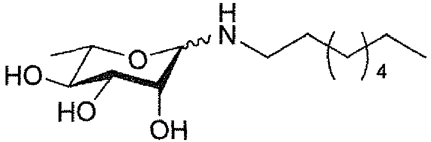

De l'octylamine (0,79 g ; 6,1 mmol) est mélangée à 1,5 g de silice (SiO₂). Les réactifs sont mélangés et broyés dans un mortier à l'aide d'un pilon pendant 2 minutes, puis 1 g de L-rhamnose monohydrate (5,5 mmol) est ajouté dans le mortier. Le mélange ainsi formé est broyé à l'aide d'un pilon pendant 2 minutes supplémentaires, puis introduit dans une jarre en inox munie de **4** billes en inox de 13 mm de diamètre. La jarre est placée dans un broyeur à billes type vibrationnel Spex 8000M et agitée pendant 1h30.

Le brut réactionnel est dissous à reflux dans 100 mL d'éthanol, puis filtré sur un fritté comportant 50 g de silice et lavé avec 50 mL d'éthanol chaud. Après évaporation de l'éthanol sous pression réduite, un solide blanc-cassé est obtenu.

Un lavage avec 10 mL de diéthyl éther à 40°C suivi d'une filtration sur fritté permet d'obtenir 1,29 g (85%) d'un composé **le** sous forme de poudre blanche.

Mélange d'isomères α, β : α/β : 93/7 ;

Isomère α : RMN ¹H (CD₃OD) : *δ* 4.04 (d, 1H, *J* = 1.1 Hz, H-1) ; 3.78 (dd, 1H, *J* = 3.3, 1.1 Hz, H-2) ; 3.40 (dd, 1H, *J* = 9.2, 3.3 Hz, H-3) ; 3.28 (t, 1H, *J* = 9.2Hz, H-4) ; 3.18 (dq, 1H, *J* = 9.2, 6.0 Hz, H-5) ; 2.91 (ddd, 1H, *J* = 11.6, 8.4, 6.4 Hz, -HN-***CH₂***-) ; 2.59 (ddd, 1H, *J* = 11.6, 8.4, 5.9 Hz, -HN-***CH₂***-) ; 1.61-1.41 (m, 2H, -NH₂-CH₂-***CH₂***-) ; 1.40-1.29 (m, 10H, -CH₂-CH₂-) ; 1.28 (d, 3H, *J* = 5.9 Hz, H-6) ; 0.90 (t, 3H, *J* = 6.8 Hz, ***CH₃***-CH₂-).

RMN ¹³C (CD₃OD) : *δ* 88.4 (C-1), 76.0 (C-3), 74.6 (C-5), 74.1 (C-4), 73.1 (C-2), 46.3 (-HN-***CH₂***-), 33.0, 31.0, 30.6, 30.4, 28.4, 23.7 (-CH₂-CH₂-), 18.1 (C-6), 14.4 (***CH₃***-CH₂-).

Spectrométrie de masse haute résolution HRMS [M+H]⁺ calculée pour C₁₄H₂₉NO₄ : *m*/*z* 276.2175, trouvée *m*/*z* 276.2182.

### Exemple 4 : Préparation d'un composé Id de formule :

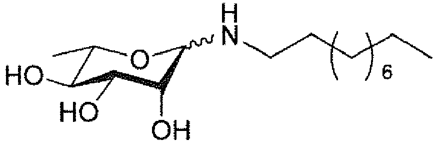

De la décylamine (1 g ; 6,6 mmol) est mélangée à 1,5 g de silice (SiO₂). Les réactifs sont ensuite mélangés et broyés dans un mortier à l'aide d'un pilon pendant 2 minutes, puis 1 g de L-rhamnose monohydrate (5,5 mmol) est ajouté dans le mortier. Le mélange ainsi formé est broyé à l'aide d'un pilon pendant 2 minutes supplémentaires, puis introduit dans une jarre en inox munie de 4 billes en inox de 13 mm de diamètre. La jarre est placée dans un broyeur à billes type vibrationnel Spex 8000M et agitée pendant lh30.

Le brut réactionnel est dissous à reflux dans 100 mL d'éthanol, puis filtré sur un fritté comportant 50 g de silice et lavé avec 50 mL d'éthanol chaud. Après évaporation de l'éthanol sous pression réduite, un solide blanc-cassé est obtenu.

Un lavage avec 10 mL de diéthyl éther à 40°C suivi d'une filtration sur fritté permet d'obtenir 1,58 g (95%) d'un composé **Id** sous forme d'une poudre blanche.

Mélange d'isomères α, β : α/β : 91/9 ;

Isomère α : RMN ¹H (CD₃OD) : *δ* 4.04 (d, 1H, *J* = 1.1 Hz, H-1) ; 3.78 (dd, 1H, *J* = 3.3, 1.1 Hz, H-2) ; 3.40 (dd, 1H, *J* = 9.2, 3.3 Hz, H-3) ; 3.28 (t, 1H, *J* = 9.2 Hz, H-4) ; 3.17 (dq, 1H, *J* = 9.1, 6.0 Hz, H-5) ; 2.91 (ddd, 1H, *J* = 11.6, 8.5, 6.5 Hz, -HN-***CH₂***-) ; 2.59 (ddd, 1H, *J* = 11.4, 8.4, 5.9 Hz, -HN-***CH₂***-) ; 1.61-1.41 (m, 2H, -NH₁-CH₂-***CH₂***-); 1.41-1.29 (m, 6H, -CH₂-CH₂-) ; 1.27 (d, 3H, *J* = 5.9 Hz, H-6) ; 0.91 (t, 3H, *J* = 6.8 Hz, ***CH₃***-CH₂-).

RMN ¹³C (CD₃OD) : *δ* 88.4 (C-1), 76.0 (C-3), 74.7 (C-5), 74.2 (C-4), 73.1 (C-2), 46.3 (-HN-***CH₂***-), 32.9, 31.0, 28.1, 23.7 (-CH₂-CH₂-), 18.1 (C-6), 14.4 (***CH₃***-CH₂-).

Spectrométrie de masse haute résolution HRMS [M+H]⁺ calculée pour C₁₂H₂₅NO₄ : *m*/*z* 248.1862, trouvée *m*/*z* 248.1864.

### Exemple 5 : Préparation d'un composé le de formule :

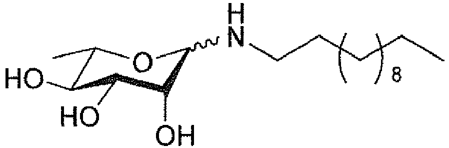

De la dodécylamine (1,18 g; 6,3 mmol) est mélangée à 1 g de silice (SiO₂). Les réactifs sont mélangés et broyés dans un mortier à l'aide d'un pilon pendant 2 minutes, puis 1 g de L-rhamnose monohydrate (5,5 mmol) est ajouté dans le mortier. Le mélange ainsi formé est broyé à l'aide d'un pilon pendant 2 minutes supplémentaires, puis est introduit dans une jarre en inox munie de 4 billes en inox de 13 mm de diamètre. La jarre est placée dans un broyeur à billes vibrationnel type Spex 8000M et agitée pendant 1h30.

Le brut réactionnel est dissous à reflux dans 100 mL d'éthanol, puis filtré sur un fritté comportant 50 g de silice et lavé avec 50 mL d'éthanol chaud. Après évaporation de l'éthanol sous pression réduite, un solide blanc est obtenu.

Un lavage avec 10 mL de diéthyl éther à 40°C permet d'obtenir 1,53g (85%) d'un composé **le** sous forme d'une poudre blanche.

Mélange d'isomères α, β : α/β : 91/9 ;

Isomère α : RMN ¹H (CD₃OD) : *δ* 4.04 (d, 1H, *J* = 0.9 Hz, H-1) ; 3.78 (dd, 1H, *J* = 3.3, 1.1 Hz, H-2) ; 3.40 (dd, 1H, *J* = 9.2, 3.3 Hz, H-3) ; 3.28 (t, 1H, *J* = 9.2 Hz, H-4) ; 3.17 (dq, 1H, *J* = 9.1, 6.0 Hz, H-5) ; 2.91 (ddd, 1H, *J* = 11.6, 8.4, 6.5 Hz, -HN-***CH₂***-) ; 2.59 (ddd, 1H, *J* = 11.6, 8.4, 6.0 Hz, -HN-***CH₂***-) ; 1.60-1.39 (m, 2H, -HN-CH₂-***CH₂***-) ; 1.39-1.19 (m, 18H,-CH₂-CH₂-) ; 1.27 (d, 3H, *J* = 6.1 Hz, H-6) ; 0.90 (t, 3H, *J* = 6.8 Hz, ***CH₃***-CH₂-).

RMN ¹³C (CD₃OD) : *δ* 88.4 (C-1), 76.0 (C-3), 74.6 (C-5), 74.2 (C-4), 73.1 (C-2), 46.3 (-HN-***CH₂***-), 33.0, 31.0, 30.7, 30.6, 30.4, 28.4, 23.7 (-CH₂-CH₂-), 18.1 (C-6), 14.4 (***CH₃***-CH₂).

Spectrométrie de masse haute résolution HRMS [M+H]⁺ calculée pour C₁₈H₃₇NO₄ : *m*/*z* 332.2801 trouvée *m*/*z* 332.2816.

### Exemple 6 : Préparation d'un composé If de formule :

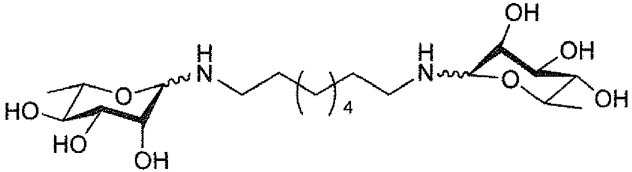

De la 1,8-diaminooctane (0,5 g ; 3,49 mmol) est mélangée à 1,26 g de L-rhamnose monohydrate (6,93 mmol) dans un mortier à l'aide d'un pilon pendant 2 minutes, puis introduit dans une jarre en inox munie de 4 billes en inox de 13 mm de diamètre. La jarre est placée dans un broyeur à billes vibrationnel type Spex 8000M et agitée pendant 3h20.

Le brut réactionnel est dissous à reflux dans 100 mL d'éthanol, puis évaporé sous pression réduite pour obtenir 1,45 g (96%) d'un composé **If** sous forme d'une poudre blanche.

Mélange d'isomères α,α β,β : α/β : 82/18 ;

Isomère α,α : RMN ¹H (DMSO-*d₆*): *δ* 4.61 (d, 2H, *J* = 4.9 Hz, OH-4) ; 4.53 (d, 2H, *J* = 5.0 Hz, OH-2), 4.50 (d, 2H, *J* = 5.7 Hz, OH-3), 3.89 (s, 2H, H-1), 3.53 (t, 2H, *J* = 3.7 Hz, H-2), 3.23-3.15 (m, 2H, H-3) ; 3.14-2.93 (m, 4H, H-4, H-5), 2.86-2.72 (m, 2H, -HN-***CH₂***-) ; 2.52-2.38 (m, 2H, -HN-***CH₂***-) ; 2.08 (s; 2H, -NH-) ; 1.45-1.30 (m, 4H, -***CH₂***-CH₂-NH-), 1.30-1.20 (m, 8H, -CH₂-CH₂-) ; 1.12 (d, 6H, *J*=6.0 Hz, H-6).

RMN ¹³C (DMSO-*d₆*) : *δ* 87.2 (C-1), 74.4 (C-3), 72.5 (C-5), 72.4 (C-4), 71.6 (C-2), 44.9 (-HN-***CH₂***-), 30.0, 29.0, 26.8 (-CH₂-CH₂-), 18.1 (C-6).

Spectrométrie de masse haute résolution HRMS [M+H]⁺ calculée pour C₂₀H₄₀N₂O₈ : *m*/*z* 437.2866 trouvée *m*/*z* 437.2872.

### Exemple 7 : Préparation d'un composé Ig de formule :

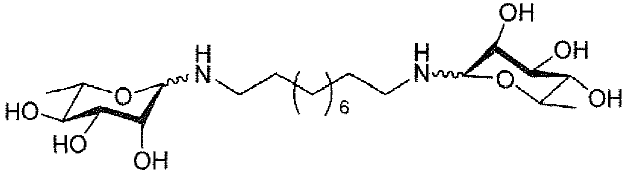

Du 1,10-diaminodécane (0,5 g; 2,9 mmol) est mélangé à 1,06 g de L-rhamnose monohydrate (5,8 mmol) dans un mortier à l'aide d'un pilon pendant 2 minutes, puis le mélange est introduit dans une jarre en inox munie de 4 billes en inox de 13 mm de diamètre. La jarre est placée dans un broyeur à billes vibrationnel type Spex 8000M et agitée pendant 3h20.

Le brut réactionnel est dissous à reflux dans 100 mL d'éthanol puis évaporé sous pression réduite pour obtenir 1,29 g (96%) du composé **If** sous forme d'une poudre blanche.

Mélange d'isomères α,α et β,β : α,α/β,β : 84/16 ;

Isomère α,α : RMN ¹H (DMSO-*d₆*) : *δ* 4.61 (d, 2H, *J* = 4.6 Hz, OH-4) ; 4.54-4.49 (m, 4H, OH-2 + OH-3) ; 3.88 (s, 2H, H-1), 3.60-3.49 (m, 2H, H-2), 3.18-3.14 (m, 2H, H-3) ; 3.10-2.93 (m, 4H, H-4, H-5), 2.84-2.70 (m, 2H, -HN-***CH₂***-) ; 2.51-2.36 (m, 2H, -HN-***CH₂***-) ; 2.08 (s, 2H, -NH-) ; 1.42-1.30 (m, 4H, -***CH₂***-CH₂-NH-), 1.29-1.19 (m, 12H, -CH₂-CH₂-), 1.12 (d, 6H, J=5.8 Hz, H-6).

RMN ¹³C (DMSO-*d₆*) : *δ* 87.2 (C-1), 74.4 (C-3), 72.5 (C-5), 72.4 (C-4), 71.6 (C-2), 44.9 (-HN-***CH₂***-), 30.0, 29.0, 26.8 (-CH₂-CH₂-), 18.1 (C-6).

Spectrométrie de masse haute résolution HRMS [M+H]⁺ calculée pour C₂₂H₄₄N₂O₈ : *m*/*z* 465.3179 trouvée *m*/*z* 465.3176.

### Exemple 8 : Préparation d'un composé Ih de formule :

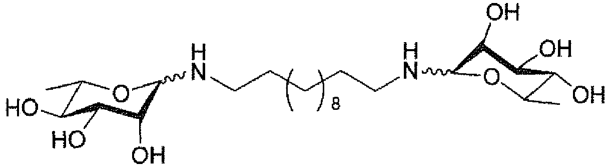

Du 1,12-diaminododécane (1 g; 5 mmol) est mélangé à 1,81 g de L-rhamnose monohydrate (10 mmol) dans un mortier à l'aide d'un pilon pendant 2 minutes. Le mélange est ensuite introduit dans une jarre en inox munie de 4 billes en inox de 13 mm de diamètre. La jarre est placée dans un broyeur à billes vibrationnel type Spex 8000M et agitée pendant 3h20.

Le brut réactionnel est dissous à reflux dans 100 mL d'éthanol, puis évaporé sous pression réduite pour obtenir 2,4 g (98%) du composé **Ih** sous forme d'une poudre blanche.

Mélange d'isomères α,α β,β : α,α/β,β : 89/11 ;

Isomère α,α : RMN ¹H (CD₃OD) : *δ* 4.04 (d, 2H, *J* = 1.1 Hz, H-1) ; 3.78 (dd, 2H, *J* = 3.3, 1.1 Hz, H-2) ; 3.40 (dd, 2H, *J* = 9.2, 3.5 Hz, H-3) ; 3.28 (t, 2H, *J* = 9.0 Hz, H-4) ; 3.17 (dq, 2H, *J* = 9.1, 6.0 Hz, H-5) ; 2.91 (ddd, 2H, *J* = 11.6, 8.4, 6.5 Hz, -HN-***CH₂***-) ; 2.59 (ddd, 2H, *J* = 11.6, 8.3, 5.9 Hz, -HN-***CH₂***-); 1.60-1.42 (m, 4H, -***CH₂***-CH₂-NH-); 1.41-1.29 (m, 16H, -CH₂-CH₂-) 1.28 (d, 6H, *J* = 5.9 Hz, H-6).

RMN ¹³C (CD₃OD) : *δ* 88.4 (C-1), 76.0 (C-3), 74.7 (C-5), 74.2 (C-4), 73,1 (C-2), 46.3 (-HN-***CH₂***-), 31.0, 30.7, 30.6, 28.4 (-CH₂-CH₂-), 18.1 (C-6).

Spectrométrie de masse haute résolution HRMS [M+H]⁺ calculée pour C₂₄H₄₈N₂O₈ : *m*/*z* 493.3513 trouvée *m*/*z* 493.3492.

### Exemple 9 : Préparation d'un composé Ii de formule :

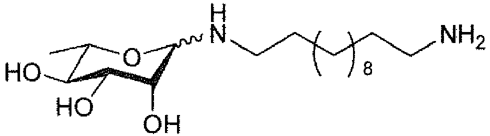

Du 1,12-diaminododécane (1g ; 5 mmol) est mélangé à 0,90 g de L-rhamnose monohydrate (4,9 mmol) dans un mortier à l'aide d'un pilon pendant 2 minutes, puis introduit dans une jarre en inox muni de 4 billes en inox de 13 mm de diamètre. La jarre est placée dans un broyeur à billes vibrationnel type Spex 8000M et agitée pendant 3h20.

Le brut est purifié par chromatographie sur gel de silice avec un mélange dichlorométhane/méthanol dont les proportions varient graduellement de 100/0 à 50/50 pour obtenir 1,31g (76%) d'un composé **Ii** sous forme d'une poudre blanche.

Mélange d'isomères α, β : α/β : 93/7 ;

Isomère α : RMN ¹H (CD₃OD) : *δ* 4.04 (d, 1H, *J* = 1.1 Hz, H-1) ; 3.78 (dd, 1H, *J* = 3.3, 0.9 Hz, H-2) ; 3.40 (dd, 1H, *J* = 9.2, 3.3 Hz, H-3) ; 3.28 (t, 1H, *J* = 9.2 Hz, H-4) ; 3.18 (dq, 1H, *J* = 9.2, 5.9 Hz, H-5) ; 2.91 (ddd, 1H, *J* = 11.6, 8.4, 6.6 Hz, -HN-***CH₂***-) ; 2.66-2.50 (m, 3H, -HN-***CH₂**-* + -***CH₂***-NH₂) ; 1.57-1.40 (m, 4H, -***CH₂***-CH₂-NH- + -***CH₂***-CH₂-NH₂) ; 1.40-1.29 (m, 16H, -CH₂-CH₂-) ; 1.27 (d, 3H, *J* = 5.9 Hz, H-6).

RMN ¹³C (CD₃OD) : *δ* 88.4 (C-1), 76,0 (C-3), 74.7 (C-5), 74.2 (C-4), 73.1 (C-2), 46.3 (-HN-***CH₂***-), 42.5 (-HN-***CH₂***-), 33.7, 31.0, 30.7, 30.6, 28.4, 28.0 (-CH₂-CH₂-), 18.1 (C-6).

Spectrométrie de masse haute résolution HRMS [M+H]⁺ calculée pour C₁₈H₃₈N₂O₄ : *m*/*z* 347.2910 trouvée *m*/*z* 347.2903.

### Exemple 10 : Préparation d'un composé Ij de formule :

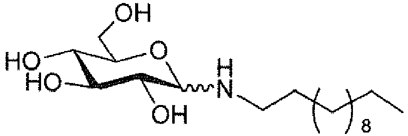

De la dodécylamine (1,13 g; 6,2 mmol) est mélangée à 1 g de silice (SiO₂). Les réactifs sont ensuite mélangés et broyés dans un mortier à l'aide d'un pilon pendant 2 minutes, puis 1 g de glucose (5,5 mmol) est rajouté dans le mortier. Le mélange ainsi formé est broyé par le pilon pendant 2 minutes supplémentaires, puis est introduit dans une jarre en inox munie de 4 billes en inox de 13 mm de diamètre. La jarre est placée dans un broyeur à billes vibrationnel type Spex 8000M et agitée pendant 1h30.

Le brut réactionnel est dissous à reflux dans 100 mL d'éthanol, puis filtré sur un fritté comportant 50 g de silice et lavé avec 50 mL d'éthanol chaud. Après évaporation de l'éthanol sous pression réduite, un solide blanc est obtenu.

Un lavage avec 10 mL de diéthyl éther à 40°C permet d'obtenir 1,56g (81%) du composé **Ij** sous forme d'une poudre blanche.

Les spectres RMN ¹H et ¹³C du composé **Ij** sont conformes à ceux décrits dans la littérature (Muhizi et al., Carbohydrate Research, 2008, 343, 2369-2375).

Mélange d'isomères α, β : le ratio n'a pas été déterminé précisément, toutefois l'isomère β est majoritaire.

Isomère β : RMN ¹H (DMSO-*d₆*) : *δ* 4.81 (d, 1H, *J* = 4.5 Hz, OH-3); 4.77 (d, 1H, *J* = 4.8 Hz, OH-4); 4.42 (d, 1H, *J* = 4.2 Hz, OH-2); 4.32 (t, 1H, *J* = 5.8 Hz, OH-6); 3.67-3.61 (m, 2H, H-1 + H-6a) ; 3.44-3.36 (m, 1H, H-6b) ; 3.11 (dt, 1H, *J* = 8.7, 4.4 Hz, H-3); 3.02-2.98 (m, 2H, H-4 + H-5) ; 2.95 (td, 1H, *J* = 8.6; 4.1 Hz, H-2) ; 2.80-2.70 (m, 1H, -HN-***CH₂***-) ; 2.52-2.44 (m, 1H, -HN-***CH₂***-) ; 2.14 (bs, 1H, NH) ; 1.42-1.32 (m, 2H, + -***CH₂***-CH₂-NH-) ; 1.29-1.17 (m, 18H, -CH₂-CH₂-) ; 0.85 (t, 3H, *J* = 6.5 Hz, ***CH₃***-CH₂-).

RMN ¹³C (DMSO-*d₆*) : *δ* 90.8 (C-1), 77.6 (C-3), 77.4 (C-5), 73.5 (C-2), 70.6 (C-4), 61.4 (C-6), 45.5 (-HN-***CH₂***-), 31.3, 30.0, 29.0, 28.7, 26.8, 22.1 (-CH₂-CH₂-), 13.9 (***CH₃***-CH₂-)

Spectrométrie de masse haute résolution HRMS [M+H]⁺ calculée pour C₁₈H₃₇NO₅ : *m*/*z* 348.2750 trouvée *m*/*z* 348.2758.

### Exemple 11 : Préparation d'un composé Ik de formule :

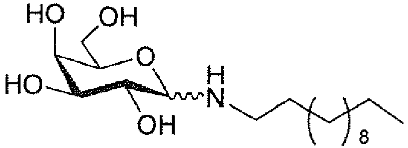

De la dodécylamine (1,13 g ; 6,2 mmol) est mélangée à 1 g de silice (SiO₂). Les réactifs sont ensuite mélangés et broyés dans un mortier à l'aide d'un pilon pendant 2 minutes. 1 g de galactose (5,5 mmol) est ajouté dans le mortier. Le mélange est broyé par le pilon pendant 2 minutes supplémentaires, puis introduit dans une jarre en inox muni de 4 billes en inox de 13 mm de diamètre. La jarre est placée dans un broyeur à billes vibrationnel type Spex 8000M et agitée pendant 1h30.

Le brut réactionnel est dissous à reflux dans 100 mL d'éthanol puis filtré sur un fritté comportant 50 g de silice et lavé avec 50 mL d'éthanol chaud. Après évaporation de l'éthanol sous pression réduite, un solide blanc est obtenu.

Un lavage avec 10 mL de diéthyl éther à 40°C permet d'obtenir 1,70g (89%) du composé **Ik** sous forme d'une poudre blanche.

Les spectres RMN ¹H et ¹³C du composé **Ik** sont conformes à ceux décrits dans la littérature (Neto et al., J. Agric. Food Chem., 2012, 60, 10516-10522).

Mélange d'isomères α, β : le ratio n'a pas été déterminé précisément, mais l'isomère β est majoritaire.

Isomère β : RMN ¹H (CD₃OD) : *δ* 3.85 (dd, 1H, *J* = 9.7, 3.6 Hz, H-4), 3.78 (d, 1H, *J* = 8.4 Hz, H-1) 3.73-3.65 (m, 2H, H-6a, H-6b), 3.49-3.31 (m, 3H, H-2, H-3, H-5), 2.97-2.79 (m, 1H, -HN-***CH₂***-), 2.68-2.52 (m, 1H, -HN-***CH₂***-), 1.58-1.41 (m, 2H, -***CH₂***-CH₂-NH-) ; 1.39-1.17 (m, 18H, -CH₂-CH₂-), 0.90 (t, 3H, *J* = 6.6 Hz, ***CH₃***-CH₂-).

RMN ¹³C (CD₃OD) : *δ* 92.5 (C-1), 77.5-75.8 (2s, C-3, C-5), 72.5 (C-2), 70.7 (C-4), 62.7 (C-6), 47.2 (-HN-***CH₂***-), 33.1, 31.2, 30.8, 30.5, 28.4, 23.7 (-CH₂-CH₂-), 14.4 (***CH₃***-CH₂-)

Spectrométrie de masse haute résolution HRMS [M+H]⁺ calculée pour C₁₈H₃₇NO₅ : *m*/*z* 348.2750 trouvée *m*/*z* 348.2757.

### Exemple 12 : Préparation d'un composé II de formule :

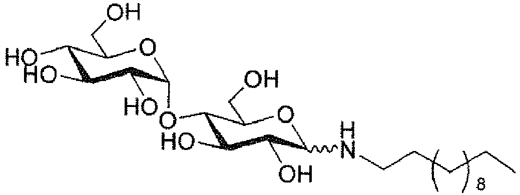

De la dodécylamine (0,56 g ; 3,05 mmol) est mélangée à 1 g de maltose monohydrate (2,77 mmol) dans un mortier à l'aide d'un pilon pendant 2 minutes, puis introduit dans une jarre en inox muni de 4 billes en inox de 13 mm de diamètre. La jarre est placée dans un broyeur à billes vibrationnel type Spex 8000M et agitée pendant 3h.

Le brut réactionnel est dissous à reflux dans 100 mL d'éthanol. Après évaporation de l'éthanol sous pression réduite, un solide blanc-cassé est obtenu.

Un lavage avec 20 mL de diéthyl éther à 40°C permet d'obtenir 1,24g (88%) du composé **II** sous forme d'une poudre beige.

Mélange d'isomères α, β : α/β : 10/90

Isomère β : RMN ¹H (CD₃OD) : *δ* 5.19 (d, 1H, *J* = 3.6 Hz, H-1') ; 3.91-3.80 (m, 4H, H-1, H-6a et H-6') ; 3.76-3.60 (m, 4H, H-3, H-3', H-5 et H-6b) ; 3.58 (t, 1H, *J* = 9.3 Hz, H-4') ; 3.50 (dd, 1H, *J* = 9.6, 3.6 Hz, H-2') ; 3.39-3.36 (m, 1H, H-5') ; 3.32 (t, 1H, *J* = 9.6 Hz , H-4) ; 3.17 (t, 1H, *J* = 8.7 Hz, H-2) ; 2.97-2.88 (m, 1H, -HN-***CH₂***-) ; 2,69-2.60 (m, 1H, -HN-***CH₂***-) ; 1.59-1.21 (m, 20H, -CH₂-CH₂-) ; 0.90 (t, 3H*, J* = 6.5 Hz, ***CH₃***-CH₂-)*.*

RMN ¹³C (CD₃OD) : *δ* 102.9 (C-1'), 91.8 (C-1), 81.6 (C-4'), 78.6 (C-3'), 77.5 (C-5'), 75.0 (C-3), 74.7 (C-5), 74.5 (C-2), 74.1 (C-2'), 71.5 (C-4), 62.7 (C-6), 62.2 (C-6'), 47.2 (-HN-***CH₂***-)*,* 33.0, 31.0, 30.7, 30.4, 28.4, 23.7 (-CH₂-CH₂-), 14.4 (***CH₃***-CH₂-).

Spectrométrie de masse haute résolution HRMS [M+H]⁺ calculée pour C₂₄H₄₇NO₁₀ : *m*/*z* 510.3249 trouvée *m*/*z* 510.3257.

### Exemple 13 : Préparation d'un composé Im de formule :

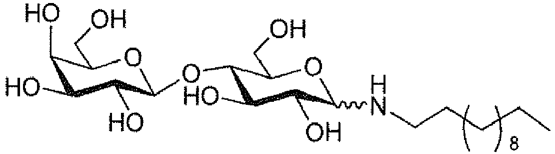

De la dodécylamine (0,56 g ; 3,05 mmol) est mélangée à 1 g de lactose monohydrate (2,77 mmol) dans un mortier à l'aide d'un pilon pendant 2 minutes, puis introduit dans une jarre en inox muni de 4 billes en inox de 13 mm de diamètre. La jarre est placée dans un broyeur à billes vibrationnel type Spex 8000M et agitée pendant 6h.

Le brut réactionnel est dissous à reflux dans 150 mL d'éthanol, puis refroidi lentement jusqu'à température ambiante. Le mélange est filtré sur Célite®. Après évaporation de l'éthanol sous pression réduite, un solide blanc est obtenu. Le procédé est repris deux fois.

Un lavage avec 20 mL de diéthyl éther à 40°C permet d'obtenir 0,77g (55%) du composé **Im** sous forme d'une poudre blanche.

Mélange d'isomères α, β : α/β : 31/69

Isomère β : RMN ¹H (Pyr-*d₅*) : *δ* 5.19 (d, 1H, *J* = 8.1 Hz, H-1') ; 4.59-4.42 (m, 5H, H-2', H-5, H-6b et H-6') ; 4.40-4.33 (m, 2H, H-1 et H-6b) ; 4.26-4.20 (m, 2H, H-3 et H-4') ; 4.17-4.09 (m, 2H, H-3' et H-4) ; 3.87-3.78 (m, 1H, H-2) ; 3.16-3.07 (m, 1H, *-*HN-***CH₂***-); 2.82-2.70 (m, 1H, -HN-***CH₂***-)*;* 1.59-1.11 (m, 20H, -CH₂-CH₂-) ; 0.86 (t, 3H, *J* = 6.6 Hz, ***CH₃***-CH₂-).

RMN ¹³C (Pyr-*d₅*) : *δ* 105.6 (C-1'), 91.8 (C-1), 82.4 (C-4'), 77.0 (2s, C-3 et C-4), 74.9 (C-3'), 74.6 (C-2), 72.2 (C-2'), 69.8 (2s, C-5 et C-5'), 62.3 (C-6'), 61.8 (C-6), 46.5 (-HN-***CH₂***-), 31.8, 30.3, 29.7, 29.4, 27.4, 22.7 (-CH₂-CH₂-), 14.0 (***CH₃***-CH₂-).

Spectrométrie de masse haute résolution HRMS [M+H]⁺ calculée pour C₂₄H₄₇NO₁₀ : *m*/*z* 510.3249 trouvée *m*/*z* 510.3272.

### Exemple 14 : Préparation d'un composé IIa de formule :

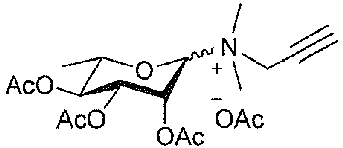

De l'iodométhane (0,88 g ; 6,2 mmol) est mélangé à 1,5 g de silice (SiO₂). 0,5 g (2,4 mmol) du composé **la** (Exemple 1) est ensuite ajouté à ce mélange, puis introduit dans une jarre en inox munie de 4 billes en inox de 13 mm de diamètre. La jarre est placée dans un broyeur à billes vibrationnel type Spex 8000M et agitée pendant 5h.

Le brut réactionnel est dissous dans 50 mL d'éthanol, puis filtré sur un fritté comportant 50 g de silice et lavé avec 50 mL d'éthanol chaud. Après évaporation de l'éthanol sous pression réduite, une huile jaune est obtenue.

Le produit brut obtenu est ensuite dissous dans 5 mL d'anhydride acétique et 0,03 g de trifluorométhanesulfonate (triflate) de cérium est ajouté. Le mélange est agité à 50°C pendant 2h. La solution est séchée sous pression réduite, puis diluée avec 30 mL d'eau. Les produits secondaires formés sont extraits 3 fois avec 30 mL d'acétate d'éthyle. La phase aqueuse est séchée par lyophilisation. Le produit obtenu est purifié par chromatographie sur colonne sur gel de silice (avec un mélange dichlorométhane/méthanol : 8/2) pour obtenir 0,43 g (49%) d'un composé **IIa** se présentant sous la forme d'une huile jaune.

Mélange d'isomères α, β : le ratio n'a pas été déterminé précisément, mais l'isomère α est majoritaire.

Isomère α : RMN ¹H (D₂O) : *δ* 5.28 (m, 1H, H-3) ; 5.21 (t, 1H, *J* = 2.3 Hz, H-4) ; 5.10 (d, 1H, *J* = 0.8 Hz, H-1) ; 4.88 (dd, 1H, *J* = 2.7, 0.8 Hz, H-2) ; 4.65-4.36 (dd, 2H, *J* = 5.8, 2.4 Hz, -N-***CH*₂**-) ; 4.13-4.02 (m, 1H, H-5) ; 3.42 (s, 3H, -N(***CH₃***)₂) ; 3.40 (m, 4H, -N(***CH₃***)₂ et ***HC*≡**C-) ; 3.06 (s, 3H, ***CH₃***CO-) ; 2.22-2.19 (3s, 9H, ***CH₃***CO-) ; 1.42 (d, 3H*, J* = 6.2 Hz, H-6).

RMN ¹³C (D₂O) : *δ* 172.6, 172.1, 172.0 (CO) ; 90.8 (C-1), 73.6 (C-5), 72.5 (C-3), 69.9 (C-3 et ***HC***≡C)*,* 64.8 (C-2), 53.7 (-N-***CH*₂**-), 48.4, 47.7 (-N(***CH₃***)₂), 41.83 (***CH₃***CO-) ; 19.9 (***CH**₃*CO-) ; 16.0 (C-6).

Spectrométrie de masse basse résolution: m/z [M]⁺ masse calculée = 356,1704 masse mesurée = 355,8

### Exemple 15 : Préparation d'un composé IIb de formule :

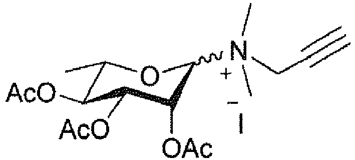

De l'iodométhane (0,81 g ; 5,7 mmol) est mélangé à 1,5 g de silice (SiO₂). 0,5 g (2,3 mmol) du composé **Ib** (Exemple 2) est ensuite ajouté à ce mélange, puis introduit dans une jarre en inox munie de 4 billes en inox de 13 mm de diamètre. La jarre est placée dans un broyeur à billes vibrationnel type Spex 8000M et agitée pendant 5h.

Le brut réactionnel est dissous dans 50 mL d'éthanol, puis filtré sur un fritté comportant 50 g de silice et lavé avec 50 mL d'éthanol chaud. Après évaporation de l'éthanol sous pression réduite, une huile incolore est obtenue.

Le produit brut obtenu est ensuite dissous dans 5 mL d'anhydride acétique et 0,03 g de trifluorométhanesulfonate (triflate) de cérium est ajouté. Le mélange est agité à 50°C pendant 2h. La solution est séchée sous pression réduite, puis diluée avec 30 mL d'eau. Les produits secondaires formés sont extraits 3 fois avec 30 mL d'acétate d'éthyle. La phase aqueuse est séchée par lyophilisation. Le produit obtenu est purifié par chromatographie sur colonne sur gel de silice (avec un mélange dichlorométhane/méthanol : 8/2) pour obtenir 0,38 g (44%) d'un composé **IIb** se présentant sous la forme d'une huile jaune-pâle.

Mélange d'isomères α, β : le ratio n'a pas été déterminé précisément, toutefois l'isomère α est majoritaire.

Isomère α : RMN ¹H (D₂O) : *δ* 6.0 (d, 1H, *J* = 2.8 Hz, H-1) ; 5.36 (dd, 1H, *J* = 2.8, 10.80 Hz, H-2) ; 5.21 (t, 1H, *J* = 10.0 Hz, H-3) ; 5.18 (m, 1H, H-4) ; 4.21-4.13 (m, 1H, H-5) ; 3.60-3.39 (m, 2H, -N-***CH₂***-) ; 3.26-3.21 (2s, 6H, -N(***CH₃***)₂) ; 2.39-2.21, 2.10 (3s, 9H, ***CH₃***-CO-) ; 1,83-1.40 (m, 4H, -CH₂-CH₂-), 1.47 (d, 3H, *J* = 6.1 Hz, H-6), 1.06 (t, 3H, *J* = 7.4 Hz, ***CH₃***-CH₂).

RMN ¹³C (D₂O) : *δ* 171.8, 171.6 (CO) ; 90.0 (C-1) ; 74.2 (C-5) ; 71.6 (C-3) ; 69.9 (C-4) ; 66.1 (C-2) ; 64.8 (-N*-****CH₂*-**) ; 49.1, 47.7 (-N-(***CH₃***)₂) ; 24.0, 19.1 (-CH₂-CH₂-) ; 20.2, 20.0, 19.7 (***CH₃***-CO)*;* 16.5 (C-6) ; 12.7 (-CH₂-***CH₃***).

Spectrométrie de masse basse résolution : m/z [M]⁺masse calculée = 374,2173 masse mesurée = 373,9

### Exemple 16 : Préparation d'un composé IIIa de formule :

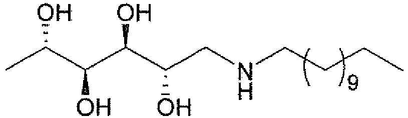

Dans une jarre en inox munie de 20 billes en inox (7 billes de 6 mm et 13 billes de 4 mm), sont introduits 0,50 g (1,5 mmol) de composé **Ie** (Exemple 5), 0,057 g (1,5 mmol) de borohydrure de sodium (NaBH₄) et 0,184 g (1,5 mmol) d'acide benzoïque. La jarre est placée dans un broyeur à billes vibrationnel type spex 8000M et agitée pendant 10 minutes. 0,1 mL d'éthanol absolu est ensuite ajouté, et la jarre est à nouveau placée dans un broyeur à billes et agitée pendant 15 minutes.

Le brut réactionnel est dissous dans 20 mL de méthanol et introduit dans un ballon de 100 mL muni d'un barreau aimanté. 1 mL d'acide chlorhydrique (37%) est ajouté et le milieu réactionnel est agité à température ambiante pendant 1h. Il se forme un précipité blanc, qui est filtré sur fritté.

Le précipité isolé est introduit dans un ballon de 100 mL muni d'un barreau aimanté avec 25 mL de méthanol. Le mélange est ensuite traité sur une résine échangeuse d'anions Amberlite IRA 402-OH⁻, à température ambiante pendant 2h. Après filtration sur fritté, le solvant est éliminé sous pression réduite pour donner 0,32 g (62%) du composé **IIIa** sous forme d'une poudre blanche.

RMN ¹H (Pyr-*d₅*) : *δ* 4.72 (d, 1H, *J* = 7.0 Hz, H-3), 4.54-4.22 (m, 2H, H-2 et H-5), 4.29 (d, 1H, *J* = 7.4 Hz, H-4), 3.41 (qd, 2H, *J* = 5.9, 11.6 Hz, -***CH₂***-NH-), 2.71-2.61 (m, 2H, - NH-***CH₂***-CH₂), 1.69 (d, 3H, *J* = 6.1 Hz, H-6), 1.50-1.15 (m, 20H, -CH₂-CH₂), 0.88 (t, 3H, *J* = 6.3 Hz, -CH₂*-**CH₃***).

RMN ¹³C (Pyr-*d₅*) : *δ* 75.3 (C-4), 74.1 (C-3), 70.5 (C-2), 69.4 (C-5), 54.3 (-***CH₂***-NH-), 50.1 (-HN-***CH*₂**-CH₂), 31.8, 30.2, 29.6, 29.3, 27.4, 22.7 (-CH₂-CH₂-), 21.4 (C-6), 14.0 (-CH₂*-**CH₃***)*.*

Spectrométrie de masse basse résolution [M+Na]⁺ calculée pour C₁₈H₃₉NO₄ : *m*/*z* 356.2879 trouvée *m*/*z* 356.2

### Exemple 17 : Préparation d'un composé IIIb de formule :

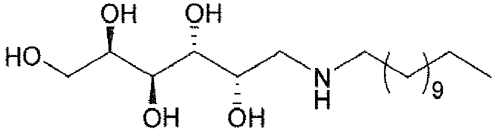

Dans une jarre en inox munie de 20 billes en inox (7 billes de 6 mm et 13 billes de 4 mm), sont introduits 0,45 g (1,29 mmol) de composé **Ij** (Exemple 10), 0,049 g (1,29 mmol) de borohydrure de sodium (NaBH₄) et 0,160 g (1,29 mmol) d'acide benzoïque. La jarre est placée dans un broyeur à billes vibrationnel type spex 8000M et agitée pendant 10 minutes. 0,2 mL d'éthanol absolu est ensuite ajouté, et la jarre est à nouveau placée dans un broyeur à billes et agitée pendant 15 minutes.

Le brut réactionnel est dissous dans 20 mL de méthanol et introduit dans un ballon de 100 mL muni d'un barreau aimanté. 1 mL d'acide chlorhydrique (37%) est ajouté et le milieu réactionnel est agité à température ambiante pendant 1h. Il se forme un précipité blanc, qui est filtré sur fritté.

Le précipité isolé est introduit dans un ballon de 100 mL muni d'un barreau aimanté avec 25 mL de méthanol. Le mélange est ensuite traité sur une résine échangeuse d'anions Amberlite IRA 402-OH⁻, à 55°C pendant 3h. Après filtration sur fritté, le solvant est éliminé sous pression réduite pour donner 0,186 g (41%) du composé **IIIb** sous forme d'une poudre blanche.

RMN ¹H (Pyr-*d₅*) : *δ* 4.76 (dd, 1H, *J* = 1.0, 4.8 Hz, H-3), 4.65-4.59 (m, 1H, H-5), 4.56-4.55 (m, 1H, H-6a), 4.54-4.47 (m, 2H, H-2 et H-4), 4.39 (dd, 1H, *J* = 5.7, 10.7 Hz, H-6b), 3.24 (qd, 2H, *J* = 4.3, 11.6 Hz, -***CH₂***-NH-), 2.67-2.51 (m, 2H, -NH-***CH₂***-CH₂), 1.50-1.15 (m, 20H, -CH₂-CH₂), 0.93 (t, 3H, *J* = 6.5 Hz, *-*CH₂-***CH₃***)*.*

RMN ¹³C (Pyr-*d₅*) : *δ* 72.6 (C-5), 72.4 (C-2 et C-4), 72.3 (C-3), 65.4 (C-6), 51.7 (-***CH₂***-NH-)*,* 50.0 (-HN-***CH₂***-CH₂), 31.8, 30.0, 29.6, 29.3, 22.7 (-CH₂-CH₂-), 14.0 (-CH₂-***CH₃***)*.*

Spectrométrie de masse basse résolution [M+Na]⁺ calculée pour C₁₈H₃₉NO₅ : *m*/*z* 372,2828 trouvée m/z 372.2

### Exemple 18 : Préparation d'un composé IIIc de formule :

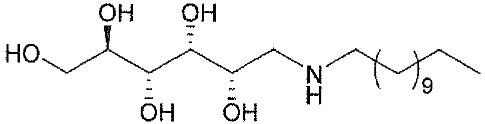

Dans une jarre en inox munie de 20 billes en inox (7 billes de 6 mm et 13 billes de 4 mm), sont introduits 0,50 g (1,44 mmol) de composé **Ik** (Exemple 11), 0,054 g (1,44 mmol) de borohydrure de sodium (NaBH₄) et 0,176 g (1,44 mmol) d'acide benzoïque, La jarre est placée dans un broyeur à billes vibrationnel type spex 8000M et agitée pendant 10 minutes. 0,2 mL d'éthanol absolu est ensuite rajouté, et la jarre est à nouveau placée dans un broyeur à billes et agitée pendant 15 minutes.

Le brut réactionnel est dissous dans 20 mL de méthanol et introduit dans un ballon de 100 mL muni d'un barreau aimanté. 1 mL d'acide chlorhydrique (37%) est ajouté et le milieu réactionnel est agité à température ambiante pendant 1h. Il se forme un précipité blanc, qui est filtré sur fritté.

Le précipité isolé est introduit dans un ballon de 100 mL muni d'un barreau aimanté avec 25 mL de méthanol. Le mélange est ensuite traité sur une résine échangeuse d'anions Amberlite IRA 402-OH⁻, à 55°C pendant 3h. Après filtration sur fritté, le solvant est éliminé sous pression réduite pour donner 0,201 g (40%) du composé **IIIc** sous forme d'une poudre blanche.

RMN ¹H (Pyr-*d₅*) : *δ* 4.86-4.82 (m, 1H, H-5), 4.72-4.66 (m, 1H, H-2), 4.63 (dd, 1H, *J* = 1.4, 8.5 Hz, H-3), 4.46 (dd, 1H, *J* = 2.2, 8.5 Hz, H-4), 4.37 (d, 2H, *J* = 5.9Hz, H-6), 3.21 (d, 2H, *J* = 5.5 Hz, -***CH₂***-NH-), 2.67-2.58 (m, 2H, -NH-***CH₂***-CH₂), 1.50-1.16 (m, 20H, -CH₂-CH₂), 0.87 (t, 3H, *J* = 6.5 Hz, -CH₂-***CH₃***).

RMN ¹³C (Pyr-*d₅*) : *δ* 74.2 (C-4), 72.0 (C-3 et C-5), 69.8 (C-2), 65.0 (C-6), 54.3 (-***CH₂***-NH-)*,* 50.2 (-HN-***CH₂***-CH₂), 31.8, 30.3, 29.6, 29.3, 27.4, 22.7 (-CH₂-CH₂-), 14.0 (-CH₂-***CH₃***)*.*

Spectrométrie de masse basse résolution [M+Na]⁺ calculée pour C₁₈H₃₉NO₅ : *m*/*z* 350,2828 trouvée *m*/*z* 350.3

### Exemple 19 : Préparation d'un composé IIId de formule :

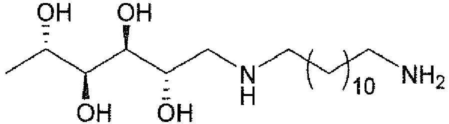

Dans une jarre en inox munie de 20 billes en inox (7 billes de 6 mm et 13 billes de 4 mm), sont introduits 0,52 g (1,51 mmol) de composé **Ii** (Exemple 9), 0,057 g (1,51 mmol) de borohydrure de sodium (NaBH₄) et 0,187 g (1,51 mmol) d'acide benzoïque. La jarre est placée dans un broyeur à billes vibrationnel type spex 8000M et agitée pendant 10 minutes. 0,2 mL d'éthanol absolu est ensuite ajouté, et la jarre est à nouveau placée dans un broyeur à billes et agitée pendant 15 minutes.

Le brut réactionnel est dissous dans 20 mL de méthanol et introduit dans un ballon de 50 mL muni d'un barreau aimanté. 1 mL d'acide chlorhydrique (37%) est ajouté et le milieu réactionnel est agité à température ambiante pendant 1h. Il se forme un précipité blanc, qui est filtré sur fritté.

Le précipité isolé est introduit dans un ballon de 100 mL muni d'un barreau aimanté avec 25 mL de méthanol. Le mélange est ensuite traité sur une résine échangeuse d'anions Amberlite IRA 402-OH⁻, à 55°C pendant 3h. Après filtration sur fritté, le solvant est éliminé sous pression réduite pour donner 0,22 g (42%) du composé **IIId** sous forme de poudre blanche.

RMN ¹H (CD₃OD) : *δ* 3.85-3.71 (m, 3H, H-2, H-3 et H-5), 3.50 (d, 1H, *J* = 7.8 Hz, H-4), 2.98-2.92 (m, 1H, -***CH₂***-NH-), 2.72-2.55 (m, 5H, *-**CH₂***-NH-*,* -NH-***CH₂***-CH₂, -CH₂-NH₂), 1.60-1.20 (m, 23H, -CH₂-CH₂- et H-6).

RMN ¹³C (CD₃OD) : *δ* 75.2 (C-4), 73.5 (C-2), 70.9 C-3), 68.7 (C-5), 53.8 (***-CH₂***-NH-), 50.6 (-HN-***CH₂***-CH₂), 42.5 (-CH₂-NH₂), 33.8, 30.7, 28.4, 28.0 (-CH₂-CH₂-), 14.0 (C-6).

Spectrométrie de masse haute résolution HRMS [M+H]⁺ calculée pour C₁₈H₄₀N₂O₄ : *m*/*z* 349,2988 trouvée *m*/*z* 349,306.

### Tests d'activité biologique des composés de formule (I) et (III) :

### I/ Activité antibactérienne

L'activité antibactérienne des dérivés *N*-alkyl-glycosyl(di)amine de formule (I) et de dérivés *N*-alkyl-glycamine de formule générale (III), synthétisés selon le procédé de l'invention a été évaluée sur trois bactéries de genres différents : *Agrobacterium tumefaciens* (Cha *et al*., 1998), *Pectobacterium atrosepticum,* et *Dickeya dianthicola.*

*Agrobacterium tumefaciens* est une alpha-protéobactéria, et *Pectobacterium atrosepticum* et *Dickeya dianthicola* sont des gamma-protéobactéria. Ce sont des bactéries rhizosphériques phytopathogènes. *Agrobacterium tumefaciens* est responsable d'une maladie appelée « galle du collet » qui se traduit par la formation d'une tumeur au site d'infection. Les bactéries du genre *Agrobacterium* sont résistantes à de nombreux antibiotiques de type bêta-lactame. *Pectobacterium* et *Dickeya* sont les agents bactériens responsables de la jambe noire et de la pourriture molle chez plusieurs plante-hôtes, principalement la pomme de terre et également d'autres plantes telles que l'endive, la betterave sucrière, la tomate ou encore la carotte.

Pour chaque molécule, la Concentration Minimale Inhibitrice (CMI), ainsi que la Concentration Minimale Bactéricide (CMB) a été évaluée.

La CMI est la plus faible concentration de molécules (en µg/mL) inhibant toute culture visible après 36h de culture à 30°C.

La CMB est la plus faible concentration de molécule (en µg/mL) laissant moins de 0,01% de survivants de l'inoculum de départ après 36h de culture à 30°C.

Pour définir la CMI et la CMB de chaque molécule, des cultures de chacune de deux souches bactériennes sont mises en place selon le protocole suivant : 20 µL d'une solution à 10⁶ UFC/mL de bactérie (*Agrobacterium tumefaciens* ou *Pectobacterium atrosepticum* ou *Dickeya dianthicola*) sont ajoutés à 10 µL de solution comprenant la molécule à tester solubilisée dans du DMSO ou du méthanol, et ajusté à un volume final de 200 µL dans du milieu TY (tryptone 5g/L et extrait de levure 3g/L). La gamme de concentration testée pour chacune des molécules est comprise entre 500 et 0,5 µg/mL. La concentration bactérienne finale est de 10⁵ UFC/puit. La CMI est déterminée après 36h d'incubation à 30°C par une lecture de densité optique à 600 nm. La CMB est déterminée par étalement de 100 µL de milieu issu de la culture précédente sur un milieu TY gélosé. La concentration la plus faible pour laquelle aucune colonie ne pousse sur la boîte est la CMB.

Deux expériences sont répétées en parallèle pour chaque échantillon.

Le 4-nitropyridine-*N*-oxide (4-NPO), produit commercial Sigma (CAS number : 1124-33-0) est utilisé comme molécule bactériostatique de référence avec une CMI de 125 µg/mL avec *Agrobacterium tumefaciens* et *Pectobacterium atrosepticum,* et de 16 µg/mL avec *Dickeya dianthicola.*

Les résultats présentés dans le **Tableau 1** ci-après correspondent aux valeurs des CMI (en µg/mL) de certains dérivés *N*-alkyl-glycosyl(di)amine de formule (I) et de certains dérivés *N*-alkyl-glycamine de formule (III) vis-à-vis des bactéries *Agrobacterium tumefaciens, Pectobacterium atrosepticum* et *Dickeya dianthicola.*

**Tableau 1 : CMI de dérivés N-alkyl-glycosyl(di)amine de formule (I) et de dérivés N-alkyl-glycamine de formule (III) (en µg.mL⁻¹)**

| | **Ic** | **Id** | **Ie** | **If** | **Ig** | **Ih** | **Ii** | **Ij** | **Ik** | **IIIa** | **IIIb** | **IIIc** | **IIId** | **4-NPO** |
|---|---|---|---|---|---|---|---|---|---|---|---|---|---|---|
| ***Pectobacterium atrosepticum*** | 62,5 | 62,5 | 16 | 250 | 125 | 62,5 | 8 | 16 | 16 | 32 | 32 | 62,5 | 62,5 | 125 |
| ***Agrobacterium tumefaciens*** | 125 | 62,5 | 4 | >500 | 125 | 62,5 | 16 | 8 | 2 | 16 | 16 | >500 | 62,5 | 125 |
| ***Dickeya dianthicola*** | n.d. | n.d. | 16 | n.d. | n.d. | n.d. | n.d. | n.d. | n.d. | 62,5 | 62,5 | >500 | 16 | 16 |

| | | | | | | | | | | | | | | |
|---|---|---|---|---|---|---|---|---|---|---|---|---|---|---|
| n.d. : valeurs non déterminées | | | | | | | | | | | | | | |

Une inhibition de la croissance des bactéries a été observée pour les composés **Ic**, **Id**, **Ie, If**, **Ig, Ih, Ii, Ij, Ik, IIIa, IIIb, IIIc et IIId** et comparée au 4-NPO qui est la molécule bactériostatique de référence, après 36h d'incubation à 30°C.

Les résultats rassemblés dans le **Tableau 2** ci-après correspondent aux valeurs des CMB (en µg/mL) de dérivés *N*-alkyl-glycosyl(di)amine de formule (I) et de dérivés *N*-alkyl-glycamine de formule (III) vis-à-vis des bactéries *Agrobacterium tumefaciens*, *Pectobacterium atrosepticum* et *Dickeya dianthicola.*

**Tableau 2 : CMB de dérivés N-alkyl-glycosyl(di)amine de formule (I) et de dérivés N-alkyl-glycamine de formule (III) (en µg.mL⁻¹)**

| | **Ic** | **Id** | **Ie** | **If** | **Ig** | **Ih** | **Ii** | **Ij** | **Ik** | **IIIa** | **IIIb** | **IIIc** | **IIId** | **4-NPO** |
|---|---|---|---|---|---|---|---|---|---|---|---|---|---|---|
| ***Pectobacterium atrosepticum*** | >500 | 250 | 16 | - | 250 | 125 | 16 | - | 32 | 32 | 32 | 62,5 | 62,5 | - |
| ***Agrobacterium tumefaciens*** | - | 250 | 8 | - | 250 | 500 | 32 | 16 | - | 62,5 | 32 | >500 | 250 | - |
| ***Dickeya dianthicola*** | n.d. | n.d. | 16 | n.d. | n.d. | n.d. | n.d. | n.d. | n.d. | 62,5 | 62,5 | >500 | 125 | 16 |

| | | | | | | | | | | | | | | |
|---|---|---|---|---|---|---|---|---|---|---|---|---|---|---|
| n.d. : valeurs non déterminées | | | | | | | | | | | | | | |

Les dérivés *N*-alkyl-glycosyl(di)amine de formule (I) et les dérivés *N*-alkyl-glycamine de formule (III) synthétisées ont montré une activité bactéricide vis-à-vis des bactéries *Agrobacterium tumefaciens, Pectobacterium atrosepticum* et *Dickeya dianthicola,* préférentiellement les composés **Id**, **Ie**, **Ig, Ih, Ii, Ij, Ik, IIIa**, **IIIb**, **IIIc** et **IIId**.

L'impact des dérivés *N*-alkyl-glycosyl(di)amine de formule (I) a été mesuré via les CMI et CMB des mélanges de composés **Ie/Ih**, **Ie/Id**, **Ih/Id** (ratios 50/50 en g/g). Le même protocole d'évaluation antibactérienne que celui décrit précédemment avec les dérivés *N-*alkyl-glycosyl(di)amine de formule (I) a été appliqué pour ces mélanges.

Les **Tableaux 3** et **4** suivants rassemblent les résultats CMI et CMB obtenus après évaluation anti-bactérienne des mélanges de composés **Ie/Ih**, **Ie/Id**, **Ih/Id**

**Tableau 3 : CMI des mélanges de composés Ie/Ih, Ie/Id, Ih/Id (en µg.mL⁻¹)**

| | **Ie/Ih** | **Ie/Id** | **Ih/Id** |
|---|---|---|---|
| ***Pectobacterium atrosepticum*** | 16 | 32 | 62,5 |
| ***Agrobacterium tumefaciens*** | 8 | 8 | 125 |

**Tableau 4 : CMB des mélanges de composés Ie/Ih, Ie/Id, Ih/Id (en µg.mL⁻¹)**

| | **Ie/Ih** | **Ie/Id** | **Ih/Id** |
|---|---|---|---|
| ***Pectobacterium atrosepticum*** | 62,5 | 62,5 | 125 |
| ***Agrobacterium tumefaciens*** | 62,5 | 32 | 125 |

Ces résultats montrent que les molécules obtenues ont des activités antibactériennes qui pourraient être exploitées pour protéger différentes plantes comme la pomme de terre contre des bactéries phytopathogènes. Par ailleurs, les associations de dérivés *N*-alkyl-glycosyl(di)amine de formule (I) montrent également un effet de synergie.

### II/ Activité antifongique

L'activité antifongique des dérivés *N*-alkyl-glycosyl(di)amine de formule (I) et des dérivés *N*-alkyl-glycamine de formule (III) synthétisés selon le procédé de l'invention a été évaluée sur deux champignons : *Fusarium roseum* et *Fusarium sambucinum.*

Pour cela, 500 µL de solution comprenant le dérivé à tester, solubilisé dans du DMSO ou du méthanol, sont mélangés à 19,5 mL de milieu gélosé malt (Sigma aldrich) permettant la croissance de ces deux isolats de *Fusarium.* Le dérivé à tester est préparé dans 2 mL de méthanol, puis mélangé à 18 mL de milieu gélosé malt (Sigma aldrich). Un spot de 10 µL de culture de chaque *Fusarium* est ensuite déposé au centre de chaque boîte de Pétri. Après 10 jours d'incubation à 20°C, les diamètres de croissance sont mesurés.

La gamme de concentrations en dérivés *N*-alkyl-glycosyl(di)amine de formule (I) testée varie de 0,1 à 25 µmοl/mL.

La croissance des *Fusarium* sur milieux contenant les différentes molécules est comparée à celle obtenue uniquement avec le DMSO ou le méthanol.

Les premiers essais ont été réalisés avec le composé **Ij** à des concentrations variant de 1 à 25 µmol/mL. 500 µL d'une solution comprenant le composé **Ij** dans du DMSO est mélangé à 19,5 mL de milieu gélosé malt (Sigma aldrich). Les résultats obtenus sont exprimés en pourcentage d'inhibition de croissance mycélienne et sont rassemblés dans le **Tableau 5.**

**Tableau 5 : Inhibition de la croissance des champignons Fusarium roseum et Fusarium sambucinum en présence de composé Ij à différentes concentrations (1 à 25 µmol/mL) (en %)**

| | **1** | **2,5** | **5** | **10** | **20** | **25** |
|---|---|---|---|---|---|---|
| ***Fusarium roseum*** | 100 | 100 | 100 | 100 | 100 | 100 |
| ***Fusarium sambucinum*** | 50 | 50 | 100 | 100 | 100 | 100 |

Le même protocole d'évaluation antifongique que celui décrit précédemment avec le composé **Ij** a été appliqué pour d'autres dérivés *N*-alkyl-glycosyl(di)amine de formule (I) et dérivés *N*-alkyl-glycamine de formule (III) à des concentrations variant de 1 à 20 µmοl/mL. Les résultats obtenus sont regroupés dans les **Tableaux 6 et 7.**

2 mL de solution comprenant le composé à tester solubilisé dans du méthanol est mélangé à 18 mL de milieu gélosé malt (Sigma aldrich) permettant la croissance de ces deux isolats de *Fusarium.* Un spot de 10 µL de culture de chaque *Fusarium* est ensuite déposé au centre de chaque boîte de Pétri. Après plus de 10 jours d'incubation à 20°C, les diamètres de croissance sont mesurés. Les pourcentages d'inhibition sont ensuite déterminés après comparaison avec la croissance mycélienne observée avec le témoin négatif (avec le méthanol et sans composé actif).

**Tableau 6 : Inhibition de la croissance des champignons Fusarium roseum en présence de dérivés N-alkyl-glycosyl(di)amine de formule (I) et de dérivés N-alkyl-glycamine de formule (III) à différentes concentrations (1 à 20 µmol/mL)**

| | **1** | **2,5** | **5** | **10** | **20** |
|---|---|---|---|---|---|
| **Ib** | 0 | 0 | 0 | 70 | 88 |
| **Ic** | 0 | 18 | 99 | 99 | 100 |
| **Ie** | 94 | 96 | 100 | 100 | 100 |
| **Ih** | 0 | 28 | 90 | 93 | 93 |
| **Ii** | 0 | 34 | 93 | 93 | 100 |
| **Ik** | 96 | 96 | 96 | n.d. | n.d. |
| **IIIa** | 100 | 100 | 100 | n.d | n.d. |
| **IIIb** | 100 | 100 | 100 | n.d | n.d |
| **IIIc** | 100 | 100 | 100 | n.d | n.d |

| | | | | | |
|---|---|---|---|---|---|
| n.d. : valeurs non déterminées | | | | | |

**Tableau 7: Inhibition de la croissance des champignons Fusarium sambucinum en présence de dérivés N-alkyl-glycosyl(di)amine de formule (I) et de dérivés N-alkyl-glycamine de formule (III) à différentes concentrations (1 à 20 µmol/mL)**

| | **1** | **2,5** | **5** | **10** | **20** |
|---|---|---|---|---|---|
| **Ib** | 0 | 0 | 4 | 76 | 90 |
| **Ic** | 10 | 33 | 89 | 90 | 100 |
| **Ie** | 95 | 96 | 100 | 100 | 100 |
| **Ih** | 12 | 27 | 100 | 100 | 100 |
| **Ii** | 28 | 60 | 100 | 100 | 100 |
| **Ik** | 96 | 96 | 96 | n.d | n.d |
| **IIIa** | 100 | 100 | 100 | n.d | n.d |
| **IIIb** | 96 | 96 | 100 | n.d | n.d |
| **IIIc** | 98 | 100 | 100 | n.d | n.d |

| | | | | | |
|---|---|---|---|---|---|
| n.d. : valeurs non déterminées | | | | | |

Ces résultats montrent que les dérivés *N*-alkyl-glycosyl(di)amine de formule (I) et de dérivés *N*-alkyl-glycamine de formule (III) ont une forte activité inhibitrice contre *Fusarium roseum* et *Fusarium sambucinum.*

Certains dérivés *N-*alkyl-glycosyl(di)amine de formule (I) et de dérivés *N*-alkyl-glycamine de formule (III) ont également fait l'objet d'essais biologiques contre *Phytophthora infestans* afin de déterminer leur activité antifongique contre cet Oomycète responsable du mildiou chez les plantes telles que la pomme de terre. Des essais consistant à mesurer leur effet inhibiteur *in vitro,* ont été menés.

Dans cette approche, les diamètres d'inhibition sont déterminées selon le protocole suivant : 20 mg de dérivé *N*-alkyl-glycosyl(di)amine de formule (I) ou de dérivé *N*-alkyl-glycamine de formule (III) est mélangée à 200 µL d'eau distillée, jusqu'à obtention d'un mélange visqueux. Quatre spots de 10 µL de ce mélange, sont déposés aux quatre extrémités de boîtes de Pétri ayant un milieu V8 agar gélosé, puis un carré de gélose contenant du mycélium de *Phytophthora infestans* est déposé au centre de la boîte. Le témoin négatif a été fait avec de l'eau distillée (sans dérivé *N*-alkyl-glycosyl(di)amine de formule (I) ou *N*-alkyl-glycamine de formule (III)) et le témoin positif avec le Ranman Top (fongicide commercial couramment utilisé pour lutter contre le mildiou). Les diamètres d'inhibition des dérivés *N-*alkyl-glycosyl(di)amine de formule (I) et des dérivés *N*-alkyl-glycamine de formule (III), et des témoins positifs et négatifs, ont été mesurés après 6 jours, et sont rassemblés dans le **Tableau 8.**

**Tableau 8 : Diamètres d'inhibition des dérivés N-alkyl-glycosyl(di)amine de formule (I) et des dérivés N-alkyl-glycamine de formule (III) vis-à-vis de Phytophthora infestans au bout de 6 jours**

| | Diamètres moyens de zone d'inhibition (mm) |
|---|---|
| **Ie** | 21 |
| **Ij** | 24 |
| **Ik** | 24 |
| **IIIa** | 24 |
| **IIIb** | 20 |
| **IIIc** | 18 |
| **IIId** | 0 |
| **Témoin négatif (eau distillée)** | 0 |
| **Témoin positif (Ranman Top)** | 35 |

Les résultats obtenus au bout de 6 jours montrent que les dérivés *N*-alkyl-glycosyl(di)amine de formule (I) et les dérivés *N*-alkyl-glycamine de formule (III) obtenus selon l'invention, ont de bonnes activités antifongiques contre l'Oomycète *Phytophthora infestans.*

## Revendications

1. Procédé de synthèse d'un dérivé *N*-alkyl-glycosyl(di)amine répondant à la formule générale (I) suivante : 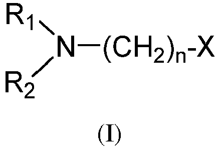 dans laquelle :
- R₁ est un radical glycosyle choisi parmi un radical rhamnosyle, glucosyle, galactosyle, maltosyle, lactosyle,
- R₂ représente un atome d'hydrogène,
- n est un nombre entier allant de 0 à 20,
- X représente un radical alkyle, alcényle, alcynyle, ou un groupe-NR₃R₄, dans lequel R₃ représente un atome d'hydrogène ou un radical glycosyle choisi parmi un radical rhamnosyle, glucosyle, galactosyle, maltosyle, lactosyle, et R₄ représente un atome d'hydrogène ou un radical alkyle en C₁-C₁₂,
à la condition que n soit différent de 0 lorsque X est un groupe -NR₃R₄,
ledit procédé comprenant les étapes suivantes :
(i) le mélange dans un réacteur d'un composé ose ou oside choisi parmi le rhamnose, le glucose, la galactose, le maltose, le lactose et d'une amine de formule R₂NH-(CH₂)ₙ-X, et
(ii) le broyage dudit mélange dans un broyeur à billes.

2. Procédé selon la revendication 1 dans lequel l'étape de réaction entre le composé ose ou oside et l'amine de formule R₂NH-(CH₂)ₙ-X se fait sans solvant.

3. Procédé selon l'une des revendications 1 ou 2 dans lequel le rapport molaire du composé ose ou oside à l'amine est compris entre 1/1 et 1/1,2.

4. Procédé selon l'une des revendications 1 à 3 dans lequel les billes du broyeur et le mélange du composé ose ou oside et de l'amine de formule R₂NH-(CH₂)ₙ-X représentent au moins 1/3 du volume du réacteur.

5. Procédé selon l'une des revendications 1 à 4 dans lequel le réacteur et/ou les billes du broyeur sont en inox, en carbure de tungstène ou en zircone.

6. Procédé selon l'une des revendications 1 à 5 dans lequel le réacteur et les billes du broyeur sont constitués du même matériau.

7. Procédé selon l'une des revendications 1 à 6 dans lequel l'étape (ii) de broyage dure au moins 1 heure, et de préférence de 1 à 4 heures.

8. Procédé selon l'une des revendications 1 à 7 dans lequel le broyeur fonctionne à une fréquence comprise entre 20 et 50 Hz, et de préférence à 50 Hz.

9. Procédé selon l'une des revendications 1 à 8 comprenant une étape préalable au cours de laquelle l'amine de formule R₂NH-(CH₂)ₙ-X est imprégnée sur un auxiliaire choisi parmi la silice (SiO₂), l'alumine (Al₂O₃), les argiles et les carbonates.

10. Procédé selon l'une des revendications 1 à 9 comprenant une étape (iii) de purification et/ou de filtration.

11. Procédé selon l'une des revendications 1 à 10 comprenant une étape (iv) de transformation du dérivé *N*-alkyl-glycosyl(di)amine de formule (I) en un sel *N*-alkyl-glycosyl(di)ammonium quaternaire par réaction avec un agent de quaternisation de formule R₅-Y, dans lequel :
- R₅ représente un atome d'hydrogène ou un radical alkyle en C₁-C₂₂, et
- Y représente un atome d'halogène, un groupe sulfate R₆O-SO₂-O- ou un groupe sulfonate R₆-SO₂-O, dans lesquels R₆ est un radical alkyle en C₁-C₁₂.

12. Procédé selon l'une des revendications 1 à 10 comprenant une étape (iv') de transformation du dérivé *N*-alkyl-glycosyl(di)amine de formule (I) en un dérivé *N-*alkyl-glycamine par broyage du dérivé *N*-alkyl-glycosyl(di)amine de formule (I) avec
- un agent de réduction qui est un borohydrure métallique choisi parmi NaBH₄, LiBH₄, NaBH₃CN, LiBH₃CN, NaBH(OAc)₃, LiBH(OAc)₃, ZnBH₄, le L-sélectride C₁₂H₂₇BLi, le diborane B₂H₆, ou un hydrure d'aluminium choisi parmi LiAlH₄, LiAlH(O-tBu)₃, l'hydrure de diisobutylaluminium C₈H₁₉Al, l'hydrure de bis(2-méthoxyéthoxy)aluminium de sodium C₆H₁₆AlNaO₄, ou un hydrure covalent choisi parmi GaH₃, NaGaBH₄, et de préférence l'agent de réduction est NaBH₄,
- et un acide, en présence d'un solvant polaire.

## Patentansprüche

1. Syntheseverfahren eines *N*-Alkyl-glycosyl(di)amin-Derivats der folgenden allgemeinen Formel (I): 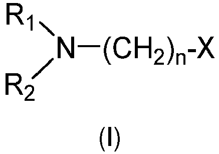 bei der:
- R₁ ein Glycosylrest ist, ausgewählt aus einem Rhamnosyl-, Glucosyl-, Galactosyl-, Maltosyl-, Lactosylrest,
- R₂ ein Wasserstoffatom darstellt,
- n eine ganze Zahl im Bereich von 0 bis 20 ist,
- X einen Alkyl-, Alkenyl-, Alkinylrest oder eine -NR₃R₄-Gruppe darstellt, in der R₃ ein Wasserstoffatom oder einen Glycosylrest darstellt, ausgewählt aus einem Rhamnosyl-, Glucosyl-, Galactosyl-, Maltosyl-, Lactosylrest, und R₄ ein Wasserstoffatom oder einen C₁-C₁₂-Alkylrest darstellt, unter der Bedingung, dass n sich von 0 unterscheidet, wenn X eine -NR₃R₄-Gruppe ist,
wobei das Verfahren die folgenden Schritte umfasst:
(i) das Mischen einer Monosaccharid- oder Osidzusammensetzung, ausgewählt aus Rhamnose, Glucose, Galactose, Maltose, Lactose, und einem Amin der Formel R₂NH-(CH₂)ₙ-X in einem Reaktor, und
(ii) das Mahlen der Mischung in einer Kugelmühle.

2. Verfahren nach Anspruch 1, wobei der Reaktionsschritt zwischen der Monosaccharid- oder Osidzusammensetzung und dem Amin der Formel R₂NH-(CH₂)ₙ-X ohne Lösemittel stattfindet.

3. Verfahren nach einem der Ansprüche 1 oder 2, wobei das molare Verhältnis der Monosaccharid- oder Osidzusammensetzung zu dem Amin zwischen 1/1 und 1/1,2 enthalten ist.

4. Verfahren nach einem der Ansprüche 1 bis 3, wobei die Kugeln der Mühle und die Mischung der Monosaccharid- oder Osidzusammensetzung und des Amins der Formel R₂NH-(CH₂)ₙ-X mindestens 1/3 des Reaktorvolumens darstellen.

5. Verfahren nach einem der Ansprüche 1 bis 4, wobei der Reaktor und/oder die Kugeln der Mühle aus Edelstahl, aus Wolframcarbid oder aus Zirkonoxid sind.

6. Verfahren nach einem der Ansprüche 1 bis 5, wobei der Reaktor und die Kugeln der Mühle aus dem gleichen Material bestehen.

7. Verfahren nach einem der Ansprüche 1 bis 6, wobei der Schritt (ii) des Mahlens mindestens 1 Stunde, und vorzugsweise 1 bis 4 Stunden dauert.

8. Verfahren nach einem der Ansprüche 1 bis 7, wobei die Mühle bei einer Frequenz betrieben wird, die zwischen 20 und 50 Hz enthalten ist, und vorzugsweise bei 50 Hz liegt.

9. Verfahren nach einem der Ansprüche 1 bis 8, umfassend einen vorhergehenden Schritt, im Laufe dessen das Amin der Formel R₂NH-(CH₂)ₙ-X auf ein Verarbeitungshilfsmittel, ausgewählt aus Siliciumdioxid (SiO₂), Aluminiumoxid (Al₂O₃), Tonerden und Carbonaten, imprägniert wird.

10. Verfahren nach einem der Ansprüche 1 bis 9, umfassend einen (iii) Reinigungs- und/oder Filtrationsschritt.

11. Verfahren nach einem der Ansprüche 1 bis 10, umfassend einen (iv) Umwandlungsschritt des *N*-Alkyl-glycosyl(di)amin-Derivats der Formel (I) in ein quaternäres *N*-Alkyl-glycosyl(di)ammoniumsalz durch Reaktion mit einem Quaternisierungsmittel der Formel R₅-Y, wobei:
- R₅ ein Wasserstoffatom oder einen C₁-C₂₂-Alkylrest darstellt, und
- Y ein Halogenatom, eine Sulfatgruppe R₆O-SO₂-O- oder eine Sulfonatgruppe R₆-SO₂-O darstellt, wobei R₆ ein C₁-C₁₂-Alkylrest ist.

12. Verfahren nach einem der Ansprüche 1 bis 10, umfassend einen (iv') Umwandlungsschritt des *N*-Alkyl-glycosyl(di)amin-Derivats der Formel (I) in ein *N*-Alkyl-glycamin durch Mahlen des *N*-Alkyl-glycosyl(di)amin-Derivats der Formel (I) mit
- einem Reduktionsmittel, das ein Metallborhydrid, ausgewählt aus NaBH₄, LiBH₄, NaBH₃CN, LiBH₃CN, NaBH(OAc)₃, LiBH(OAc)₃, ZnBH₄, L-Selektrid C₁₂H₂₇BLi, Diboran B₂H₆, oder ein Aluminiumhydrid, ausgewählt aus LiAlH₄, LiAlH(O-tBu)₃, Diisobutylaluminiumhydrid C₈H₁₉Al, Natrium-bis(2-methoxyethoxy)aluminiumhydrid C₆H₁₆AlNaO₄, oder ein kovalentes Hydrid, ausgewählt aus GaH₃, NaGaBH₄ ist und wobei das Reduktionsmittel vorzugsweise NaBH₄ ist,
- und einer Säure in Gegenwart eines polaren Lösemittels.

## Claims

1. Method for synthesizing an *N*-alkyl-glycosyl(di)amine derivative represented by the following general formula (I): 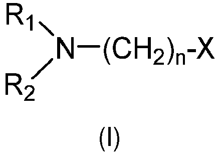 wherein:
- R₁ is a glycosyl radical chosen from a rhamnosyl, glucosyl, galactosyl, maltosyl, lactosyl radical,
- R₂ represents a hydrogen atom,
- n is an integer ranging from 0 to 20,
- X represents an alkyl, alkenyl, alkynyl radical, or an -NR₃R₄ group, wherein R₃ represents a hydrogen atom or a glycosyl radical chosen from a rhamnosyl, glucosyl, galactosyl, maltosyl, lactosyl radical, and R₄ represents a hydrogen atom or a C₁-C₁₂ alkyl radical,
with the condition that n is different from 0 when X is an -NR₃R₄ group,
said method comprising the following steps:
(i) mixing in a reactor of an ose or oside compound chosen from rhamnose, glucose, galactose, maltose, lactose and an amine of formula R₂NH-(CH₂)ₙ-X, and
(ii) grinding said mixture in a ball mill.

2. Method according to claim 1, wherein the reaction step between the ose or oside compound and the amine of formula R₂NH-(CH₂)ₙ-X is carried out without solvent.

3. Method according to any of claims 1 or 2 wherein the molar ratio of the ose or oside compound to the amine is between 1/1 and 1/1.2.

4. Method according to any of claims 1 to 3, wherein the balls of the mill and the mixture of the ose or oside compound and the amine of formula R₂NH-(CH₂)ₙ-X represent at least 1/3 of the volume of the reactor.

5. Method according to any of claims 1 to 4, wherein the reactor and/or the balls of the mill are made of stainless steel, tungsten carbide or zirconia.

6. Method according to any of claims 1 to 5, wherein the reactor and the balls of the mill are made of the same material.

7. Method according to any of claims 1 to 6, wherein the step (ii) of grinding lasts at least 1 hour, and preferably from 1 to 4 hours.

8. Method according to any of claims 1 to 7, wherein the mill operates at a frequency between 20 and 50 Hz, and preferably at 50 Hz.

9. Method according to any of claims 1 to 8, comprising a prior step during which the amine of formula R₂NH-(CH₂)ₙ-X is impregnated on an auxiliary chosen from silica (SiO₂), alumina (Al₂O₃), clays and carbonates.

10. Method according to any of claims 1 to 9, comprising a step (iii) of purification and/or filtration.

11. Method according to anyc of claims 1 to 10, comprising a step (iv) of transformation of the *N*-alkyl-glycosyl(di)amine derivative of formula (I) into a *N*-alkyl-glycosyl(di)ammonium quaternary salt by reaction with a quaternizing agent of formula R₅-Y, wherein:
- R₅ represents a hydrogen atom or a C₁-C₂₂ alkyl radical , and
- Y represents a halogen atom, a sulfate group R₆O-SO₂-O- or a sulfonate group R₆-SO₂-O, wherein R₆ is a C₁-C₁₂ alkyl radical.

12. Method according to one of claims 1 to 10, comprising a step (iv') of transformation of the *N*-alkyl-glycosyl(di)amine derivative of formula (I) into a *N*-alkyl-glycamine derivative by grinding of the *N*-alkyl-glycosyl(di)amine derivative of formula (I) with
- a reducing agent which is a metal borohydride chosen from NaBH₄, LiBH₄, NaBH₃CN, LiBH₃CN, NaBH(OAc)₃, LiBH(OAc)₃, ZnBH₄, L-selectride C₁₂H₂₇BLi, diborane B₂H₆, or an aluminium hydride chosen from LiAlH₄, LiAlH(O-tBu)₃, diisobutylaluminium hydride C₈H₁₉Al, sodium bis(2-methoxyethoxy)aluminium hydride C₆H₁₆AlNaO₄, or a covalent hydride chosen from GaH₃, NaGaBH₄, and preferably the reducing agent is NaBH₄,
- and an acid, in the presence of a polar solvent.
